# EUROPEAN PATENT APPLICATION

(11) **EP 4 368 622 A1**
(43) Date of publication of application: **15.05.2024**
(21) Application number: 22386078.4
(22) Date of filing: 10.11.2022
(51) Int. Cl.: C07D 487/04, A61K 31/4162, A61K 51/00

(54) **METHOD OF PURIFYING A COMPOUND**

(71) Applicant: AC Immune SA, 1015 Lausanne (CH)
(72) Inventor: Dimitrakopoulos, Yannis, 1030 Bussigny (CH); Molette, Jerome, 01280 Prevessin Moens (FR)
(74) Representative: Vossius & Partner Patentanwälte Rechtsanwälte mbB

(57) **Abstract**

The present invention relates to method of purifying a compound of formula (IV-F) 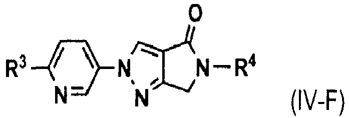 or a stereoisomer, racemic mixture, pharmaceutically acceptable salt, hydrate, or solvate thereof which contains C₁₋₄ alkyl sulfonate as a leaving group.

The compound of formula (IV-F) can be used as a precursor of an ¹⁸F-labelled agent for imaging of alpha-synuclein aggregates and determining an amount thereof. The ¹⁸F-labelled agent can be used for diagnosing a disease, disorder or abnormality associated with an alpha-synuclein aggregates (such as multiple system atrophy (MSA)) determining a predisposition to such a disease, disorder or abnormality, prognosing such a disease, disorder or abnormality, monitoring the evolution of the disease in a patient suffering from such a disease, disorder or abnormality, monitoring the progression of such a disease, disorder or abnormality and predicting responsiveness of a patient suffering from such a disease, disorder or abnormality to a treatment thereof.

## Description

### FIELD OF THE INVENTION

The present invention relates to a method of purifying a compound of formula (IV-F). The present invention also relates to a kit for preparing a radiopharmaceutical preparation, and a method of preparing a detectably labelled compound of formula (III-F). The compound of formula (III-F) can be employed in the imaging of alpha-synuclein aggregates and determining an amount thereof. Furthermore, the compounds of formula (III-F) can be used for diagnosing a disease, disorder or abnormality associated with an alpha-synuclein (a-synuclein, A-synuclein, aSynuclein, A-syn, α-syn, aSyn, a-syn) aggregates (such as multiple system atrophy (MSA)) determining a predisposition to such a disease, disorder or abnormality, prognosing such a disease, disorder or abnormality, monitoring the evolution of the disease in a patient suffering from such a disease, disorder or abnormality, monitoring the progression of such a disease, disorder or abnormality and predicting responsiveness of a patient suffering from such a disease, disorder or abnormality to a treatment thereof.

### BACKGROUND OF THE INVENTION

Many diseases of aging are based on or associated with extracellular or intracellular deposits of amyloid or amyloid-like proteins that contribute to the pathogenesis as well as to the progression of the disease. The best characterized amyloid protein that forms extracellular aggregates is amyloid beta (Abeta or Aβ).

Amyloid-like proteins that form mainly intracellular aggregates include, but are not limited to, Tau, alpha-synuclein, and huntingtin (HTT). Diseases involving alpha-synuclein aggregates are generally listed as synucleinopathies (or α-synudeinopathies) and these include, but are not limited to, Parkinson's disease (PD) and multiple system atrophy (MSA). Synucleinopathies with primarily neuronal aggregates include, but are not limited to, Parkinson's disease (sporadic, familial with SNCA (the gene encoding for the alpha-synuclein protein) mutations or SNCA gene duplication or triplication, familial with mutations in other genes than SNCA, pure autonomic failure and Lewy body dysphagia), SNCA duplication carrier, Lewy Body dementia (LBD), dementia with Lewy bodies (DLB) ("pure" Lewy body dementia), Parkinson's disease dementia (PDD), diffuse Lewy body disease (DLBD), Alzheimer's disease, sporadic Alzheimer's disease, familial Alzheimer's disease with APP mutations, familial Alzheimer's disease with PS-1, PS-2 or other mutations, familial British dementia, Lewy body variant of Alzheimer's disease and normal aging in Down syndrome. Synucleinopathies with neuronal and glial aggregates of alpha synuclein include but are not limited to multiple system atrophy (MSA) (Shy-Drager syndrome, striatonigral degeneration and olivopontocerebellar atrophy). Other diseases that may have alpha-synuclein-immunoreactive lesionsare, but are not limited to, traumatic brain injury, chronic traumatic encephalopathy, dementia puglistica, tauopathies (Pick's disease, frontotemporal dementia, progressive supranuclear palsy, corticobasal degeneration and Niemann-Pick type C1 disease, frontotemporal dementia with Parkinsonism linked to chromosome 17), motor neuron disease, Huntington's disease, amyotrophic lateral sclerosis (sporadic, familial and ALS-dementia complex of Guam), neuroaxonal dystrophy, neurodegeneration with brain iron accumulation type 1 (Hallervorden-Spatz syndrome), prion diseases, Creutzfeldt-Jakob disease, ataxia telangiectatica, Meige's syndrome, subacute sclerosing panencephalitis, Gerstmann-Straussler-Scheinker disease, inclusion-body myositis, Gaucher disease, Krabbe disease as well as other lysosomal storage disorders (including Kufor-Rakeb syndrome and Sanfilippo syndrome) and rapid eye movement (REM) sleep behavior disorder (Jellinger, Mov Disord 2003, 18 Suppl. 6, S2-12; Galvin et al. JAMA Neurology 2001, 58 (2), 186-190; Kovari et al., Acta Neuropathol. 2007, 114(3), 295-8; Saito et al., J Neuropathol Exp Neurol. 2004, 63(4), 323-328; McKee et al., Brain, 2013, 136(Pt 1), 43-64; Puschmann et al., Parkinsonism Relat Disord 2012, 18S1, S24-S27; Usenovic et al., J Neurosci. 2012, 32(12), 4240-4246; Winder-Rhodes et al., Mov Disord. 2012, 27(2), 312-315; Ferman et al., J Int Neuropsychol Soc. 2002, 8(7), 907-914; Smith et al., J Pathol. 2014;232:509-521, Lippa et al., Ann Neurol. 1999 Mar;45(3):353-7; Schmitz et al., Mol Neurobiol. 2018 Aug 22; Charles et al., Neurosci Lett. 2000 Jul 28;289(1):29-32; Wilhelmsen et al., Arch Neurol. 2004 Mar;61(3):398-406; Yamaguchi et al., J Neuropathol Exp Neurol. 2004, 80th annual meeting, vol.63; Askanas et al., J Neuropathol Exp Neurol. 2000 Jul;59(7):592-8).

Alpha-synuclein is a 140 amino acid natively unfolded protein (Iwai et al., Biochemistry 1995, 34(32), 10139-10145). The sequence of alpha-synuclein can be divided into three main domains: 1) the N-terminal region comprising of residues 1-60, which contains the 11-mer amphipatic imperfect repeat residues with highly conserved hexamer (KTKEGV). This region has been implicated in regulating alpha-synuclein binding to membranes and its internalization; 2) the hydrophobic Non Amyloid beta Component (NAC) domain spanning residues 61-95; which is essential for alpha-synuclein fibrillization; and 3) the C-terminal region spanning residues 96-140 which is highly acidic and proline-rich and has no distinct structural propensity. Parkinson's disease (PD) is the most common neurodegenerative motor disorder. The pathogenesis of PD remains elusive. However, growing evidence suggests a role for the pathogenic folding of the alpha-synuclein protein that leads to the formation of amyloid-like fibrils. Indeed, the hallmarks of PD are the presence of intracellular alpha-synuclein aggregate structures called Lewy Bodies and neurites mainly in the nigral neurons, as well as the death of dopaminergic neurons in the substantia nigra and elsewhere. Alpha-synuclein is a natively unfolded presynaptic protein that can misfold and aggregate into larger oligomeric and fibrillar forms which are linked to the pathogenesis of PD. Recent studies have implicated small soluble oligomeric and protofibrillar forms of alpha-synuclein as the most neurotoxic species (Lashuel et al., J. Mol. Biol., 2002, 322, 1089-102).

Besides Parkinson's disease, the accumulation of aggregated alpha-synuclein into Lewy bodies is a characteristic of all Lewy body diseases, including Parkinson's disease with dementia (PDD), and dementia with Lewy bodies (DLB) (Capouch et al., Neurol Ther. 2018, 7, 249-263). In DLB, Lewy Bodies are diffusely distributed throughout the cortices of the brain and in addition to Lewy Bodies and neurites, more threads and dot-like structures (Lewy dots) were found to be immunopositive for a-syn phosphorylated at Ser-129 (Outeiro et al., Mol Neurodegener. 2019, 14, 5).

Alpha-synuclein agggregates are also found in multiple system atrophy (MSA). MSA is a rare and sporadic neurodegenerative disorder that manifests with rapidly progressive autonomic and motor dysfunction, as well as variable cognitive decline. Such disorders include Shy-Drager syndrome, striatonigral degeneration and olivopontocerebellar atrophy. The disease can be clinically subclassified in parkinsonian (MSA-P) or cerebellar (MSA-C) variant, depending on the predominant motor phenotype (Fanciulli et al., N Engl J Med 2015; 372, 249-63). It is characterized by the aggregation of alpha-synuclein in the cytoplasm of oligodendrocytes, forming glial cytoplasmic inclusions (GCIs). GCIs, consisting primarily of fibrillary forms of a-synuclein, are the neuropathological hallmark of MSA and are found throughout the neocortex, hippocampus, brainstem, spinal cord and dorsal root ganglia (Galvin et al., Arch Neurol. 2001, 58,186-90). GCIs are considered a central player in the pathogenesis of MSA. A correlation between the GCI load and the degree of neuronal loss has been reported in both the striatonigral and the olivopontocerebellar regions (Stefanova et al., Neuropathol Appl Neurobiol. 2016, 42, 20-32).

The ability to image alpha-synuclein deposition in the brain would be a huge achievement for alpha-synucleopathies research, including Parkinson's disease and MSA research, diagnosis, and drug development. The accumulation of aggregated alpha-synuclein in the brain is considered a key pathological hallmark of PD and MSA and can start many years before the appearance of the symptoms. Therefore, alpha-synuclein is a priority target for drug development given not only its likely contribution to neurodegeneration but also because it can offer the possibility to treat the disease while still in the asymptomatic or prodromal stages. *In vivo* imaging of alpha-synuclein pathology could be useful as a biomarker to (i) detect the presence of the disease potentially in early stages, (ii) to evaluate disease progression and (iii) to be used as a pharmacodynamics tool for drug development. The development of an alpha-synuclein PET imaging agent is considered nowadays key for an accurate diagnosis of synucleinopathies as well as to support the clinical development of therapeutics targeting alpha-synuclein, starting from the optimal selection of the trial population (Eberling, Dave and Frasier, J. Parkinson's Disease, 3, 565-567 (2013)).

Only recently, the first non-invasive images of pathological alpha-synuclein (a-syn) in human brain were reported and presented positive clinical proof-of-concept data for an a-syn positron emission tomography (PET) tracer, ACI-12589, as an imaging agent to identify MSA patients (Capotosti F.; Discovery of [18F] ACI-12589, a novel and promising PET-tracer for alpha-synuclein; Oral presentation; ADPD 2022 International Conference; Barcelona, Spain; March 18, 2022; Smith R.; Initial scans using [18F] ACI-12589, a novel PET-tracer for alpha-synuclein; Oral presentation; ADPD 2022 International Conference; Barcelona, Spain; March 18, 2022).

Therefore, there is a clear need to find molecular probes with high alpha-synuclein selectivity which recognize and bind to the pathological alpha-synuclein. In order to reduce background signal interference resulting from non-specific off-target binding and to reduce dosing requirements, alpha-synuclein imaging compounds should bind with high affinity and selectivity to their target. For imaging of alpha-synuclein aggregates associated with neurological diseases such as multiple system atrophy (MSA), imaging compounds need to penetrate the blood brain barrier and pass into the relevant regions of the brain. For targeting intracellular amyloid-like inclusions such as alpha-synuclein, cell permeability is a further requirement of imaging compounds. A further prerequisite in order to avoid unnecessary accumulation of the compound which may result in increased risk of unwanted side-effects is a fast compound wash-out from the brain (or other targeting organ).

WO 2021/224489 discloses a new class of compounds of formula (I) which are capable of binding to alpha-synuclein. Thus, the compounds qualify as a PET tracer for the imaging of pathological alpha-syn aggregates in PD and other alpha-synucleinopathies when the compounds are radiolabelled with suitable radioisotopes, particularly ¹⁸F. The precursors of the PET tracers were purified by flash chromatography. This method has a low yield and therefore is not suitable for industrial scale production.

Due to the short half-life of ¹⁸F (approx. 110 min) the radiolabelled compounds are typically provided to the users, such as radiopharmacies, in the form of a precursor which is reacted with a ¹⁸F-fluorinating agent shortly before use. It has been observed that the precursor is unstable under normal storage conditions. An object of the present invention is thus the provision of a method of preparing a precursor compound which has improved stability. A further object of the present invention is the provision of a method which results in a low amount of impurities and is suitable for production of the precursor for diagnositic applications on an industrial scale.

### SUMMARY OF THE INVENTION

In a first aspect, the invention is directed to a method of purifying a compound of formula (IV-F) 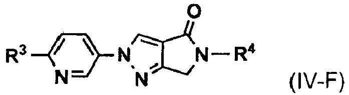 as defined herein, or a stereoisomer, racemic mixture, pharmaceutically acceptable salt, hydrate, or solvate thereof, wherein the method comprises
(i) dissolving a crude compound of formula (IV-F) in DMSO and ethanol to obtain a solution; and
(ii) conducting crystallization from the solution to obtain a purified compound of formula (IV-F).

In a second aspect, the invention relates to a compound obtainable by the method according to the first aspect.

A third aspect of the invention refers to a kit for preparing a radiopharmaceutical preparation, wherein the kit comprises a sealed vial containing at least one compound obtainable by the method according to the first aspect.

A fourth aspect of the invention relates to a method of preparing a detectably labelled compound of formula (III-F) 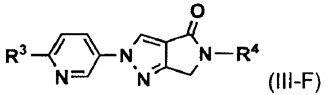
as defined herein, or a stereoisomer, racemic mixture, pharmaceutically acceptable salt, hydrate, or solvate thereof,
wherein the method comprises
reacting the compound obtainable by the method according to the first aspect with a ¹⁸F-fluorinating agent, so that LG is replaced by ¹⁸F.

### DEFINITIONS

For the purpose of interpreting this specification, the following definitions will apply unless specified otherwise, and when appropriate, terms used in the singular will also include the plural and vice versa.

"Alkyl" refers to a saturated straight or branched organic moiety consisting of carbon and hydrogen atoms. The alkyl group typically does not contain any saturation, and is usually attached to the rest of the molecule by a single bond. Examples of suitable alkyl groups have 1 to 6 carbon atoms, preferably 1 to 4 carbon atoms. The term "C₁-C₄alkyl" is to be construed accordingly. Examples of "C₁-C₄alkyl" include, but are not limited to, methyl, ethyl, propyl, isopropyl, 1-methylethyl, n-butyl, t-butyl and isobutyl, such as methyl, ethyl, propyl, isopropyl, n-butyl, t-butyl and isobutyl.

"C₁-C₄alkoxy" refers to a radical of the formula -ORa where Ra is a C₁-C₄alkyl radical as generally defined above. Examples of C₁-C₄alkoxy include, but are not limited to, methoxy, ethoxy, propoxy, isopropoxy, butoxy, and isobutoxy.

"halogenC₁-C₄alkyl" or "haloC₁-C₄alkyl" refer to C₁-C₄alkyl radical, as defined above, substituted by one or more halo radicals, as defined below. Examples of "haloC₁-C₄alkyl" include, but are not limited to, trifluoromethyl, difluoromethyl, fluoromethyl, trichloromethyl, 2,2,2-trifluoroethyl, 1,3-dibromopropan-2-yl, 3-bromo-2-fluoropropyl and 1,4,4-trifluorobutan-2-yl.

"C₃-C₆cycloalkyl" refers to a stable monocyclic saturated hydrocarbon radical consisting solely of carbon, and hydrogen atoms, having from three to six carbon atoms. Examples of C₃-C₆cycloalkyl include, but are not limited to, cyclopropyl, cyclobutyl, cyclopentyl, and cyclohexyl.

"Heterocyclyl" refers to a stable 4- to 6-membered non-aromatic monocyclic ring radical which comprises 1 or 2 heteroatoms which are, e.g., selected from N, O or S. The heterocyclyl group can be unsaturated or saturated. The heterocyclyl radical may be bonded via a carbon atom or heteroatom. Examples include, but are not limited to, azetidinyl, oxetanyl, pyrrolinyl, pyrrolidyl, tetrahydrofuryl, tetrahydrothienyl, piperidyl, piperazinyl, tetrahydropyranyl, morpholinyl or perhydroazepinyl. Examples of preferred heterocyclyl groups include, but are not limited to, azetidinyl, morpholinyl, piperazinyl, pyrrolidinyl, or piperidinyl.

"Aryl" refers to homocyclic aromatic organic moieties (for example containing 1 or 2 rings) consisting of carbon and hydrogen atoms which preferably have 5 to 12 carbon atoms, preferably 6 to 12 carbon atoms, more preferably 6 to 10 carbon atoms, yet more preferably 5 to 10 carbon atoms, even more preferably 5 or 6 carbon atoms. Examples inlcude, but are not limited to, phenyl, biphenyl, and naphthyl.

"Heteroaryl" refers to an aryl group as defined above in which at least one of the carbon atoms has been replaced by a heteroatom which is, e.g., selected from N, O or S, or heteroatom (e.g., N, O and/or S)-containing moiety. Typically the heteroaryl is a 5- to 8-membered ring system, preferably to a 5 to 6 membered ring system, in which at least one of the carbon atoms has been replaced by a heteroatom which is, e.g., selected from N, O or S. Examples of possible heteroaryl groups include, but are not limited to, furyl, pyrrolyl, thienyl, pyrazolyl, imidazolyl, thiazolyl, isothiazolyl, oxazolyl, isoxazolyl, triazolyl, tetrazolyl, pyrazinyl, pyridazinyl, pyrimidyl or pyridyl. Preferred examples thereof include pyridine, pyrazole, etc., more preferably pyridine.

"Hal" or "halogen" or "Halo" refers to F, Cl, Br, and I. With respect to diagnostic and pharmaceutical applications, F (e.g., ¹⁹F and ¹⁸F) is particularly preferred.

Unless defined otherwise, the term "leaving group" (LG) as employed herein is any leaving group and means an atom or group of atoms that can be replaced by another atom or group of atoms. Examples are given e.g. in Synthesis (1982), p. 85-125, table 2, Carey and Sundberg, Organische Synthese, (1995), page 279-281, table 5.8; or Netscher, Recent Res. Dev. Org. Chem., 2003, 7, 71-83, schemes 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp. 15-50, explicitly: scheme 4 pp. 25, scheme 5 pp 28, table 4 pp 30, Figure 7 pp 33). Preferably, the "leaving group" (LG) is selected from halogen, C₁₋₄ alkyl sulfonate and C₆₋₁₀ aryl sulfonate, wherein the C₆₋₁₀ aryl can be optionally substituted by -CH₃ or -NO₂. In the moiety R³ the leaving group LG is C₁₋₄ alkyl sulfonate.

Compounds of the formula (III-F) and their precursors such as compounds of the formula (IV-F) having one or more optically active carbons can exist as racemates and racemic mixtures, stereoisomers (including diastereomeric mixtures and individual diastereomers, enantiomeric mixtures and single enantiomers, mixtures of conformers and single conformers), tautomers, atropisomers, and rotamers. All isomeric forms are included in the present invention. Compounds described in this specification containing olefinic double bonds include E and Z geometric isomers.

Also included in this invention are all salt forms, polymorphs, hydrates and solvates (such as ethanolates).

"Pharmaceutically acceptable salts" are defined as derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as, but not limited to, hydrochloric, hydrobromic, sulfuric, sulfamic, phosphoric, nitric and the like; and the salts prepared from organic acids such as, but not limited to, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the compounds of the formula (III-F) and their precursors can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Organic solvents include, but are not limited to, nonaqueous media like ethers, ethyl acetate, ethanol, isopropanol, or acetonitrile. Lists of suitable salts can be found in Remington's Pharmaceutical Sciences, 18th ed., Mack Publishing Company, Easton, PA, 1990, p. 1445, the disclosure of which is hereby incorporated by reference.

"Pharmaceutically acceptable" is defined as those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The compounds of the formula (III-F) can also be provided in the form of a prodrug, namely a compound which is metabolized *in vivo* to the active metabolite.

The patients or subjects in the present invention are typically animals, particularly mammals, more particularly humans.

Alpha-synuclein aggregates are multimeric beta-sheet rich assemblies of alpha-synuclein monomers that can form either soluble oligomers or soluble/insoluble protofibrils or mature fibrils which coalesce into intracellular deposits detected in Parkinson's disease, multiple system atrophy (MSA) and other synucleinopathies. Alpha-synuclein aggregates that are composing Lewy pathologies can be detected as having the following morphologies: Lewy bodies, Lewy neurites, premature Lewy bodies or pale bodies, perikaryal deposits with diffuse, granular, punctate or pleomorphic patterns. Moreover, alpha-synuclein aggregates are the major component of intracellular fibrillary inclusions detected in oligodendrocytes (also referred to as glial cytoplasmic inclusions) and in neuronal somata, axons and nuclei (referred to as neuronal cytoplasmic inclusions) that are the histological hallmarks of multiple system atrophy. Alpha-synuclein aggregates in Lewy pathologies and glial cytoplasmic inclusions often display substantial increase in post-translational modifications such as phosphorylation, ubiquitination, nitration, and truncation.

Lewy bodies are abnormal aggregates of protein that develop inside nerve cells Parkinson's disease (PD), Lewy body dementia and other synucleinopathies. Lewy bodies appear as spherical masses that displace other cell components. Morphologically, Lewy bodies can be classified as being brainstem or cortical type. Classic brainstem Lewy bodies are eosinophilic cytoplasmic inclusions consisting of a dense core surrounded by a halo of 5-10-nm-wide radiating fibrils, the primary structural component of which is alpha-synuclein; cortical Lewy bodies differ by lacking a halo. The presence of Lewy bodies is a hallmark Parkinson's disease.

Lewy neurites are abnormal neuronal processes in diseased neurons containing granular material, abnormal alpha-synuclein (a-syn) filaments similar to those found in Lewy bodies, dot-like, varicose structures and axonal spheroids. Lewy neurites are a feature of synucleinopathies such as dementia with Lewy bodies, Parkinson's disease, and multiple system atrophy (MSA).

Glial cytoplasmic inclusions (GCIs or Papp-Lantos bodies) are argyrophilic cytoplasmic aggregates in oligodentroglial cells composed of filamentous alpha-synuclein. Morphologically they appear as triangles, half-moon or sickle shapes. In MSA, besides GCIs, inclusions composed of alpha-synuclein filaments are detected in neurons in the cytoplasm or beneath the nuclear membrane termed neuronal cytoplasmic inclusions and neuronal nuclear inclusions respectively. GCIs are recognized as the defining morphological feature of MSA; their widespread distribution is a criterion for the definite post-mortem neuropathological diagnosis of MSA.

The terms "disease", "disorder" or "abnormality" are used interchangeably herein.

The compounds of formula (III-F), or a stereoisomer, racemic mixture, pharmaceutically acceptable salt, hydrate, or solvate thereof, can bind to alpha-synuclein aggregates. The type of bonding between the compounds of formula (III-F), or a stereoisomer, racemic mixture, pharmaceutically acceptable salt, hydrate, or solvate thereof, has not been elucidated and any type of bonding is covered by the present invention. The wording "compound bound to the alpha-synuclein aggregates", "compound/(alpha-synuclein aggregates) complex", compound/alpha-synuclein aggregate complex", "compound/protein aggregate complex" and the like are used interchangeably herein and are not considered to be limited to any specific type of bonding.

The preferred definitions given in the "Definition"-section apply to all of the embodiments described below unless stated otherwise. Various embodiments of the invention are described herein, it will be recognized that features specified in each embodiment may be combined with other specified features to provide further embodiments of the present invention.

### BRIEF DESCRIPTION OF THE FIGURE

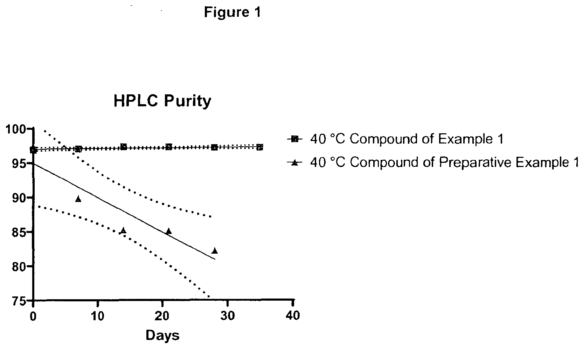

**Figure 1** shows the purity profile of the crude compound of Preparative Example 1 and of the purified compound of Example 1 over time.

### DETAILED DESCRIPTION OF THE INVENTION

The present invention relates to a method of purifying a compound of formula (IV-F) 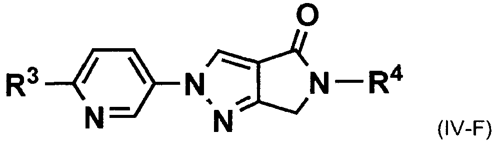 or a stereoisomer, racemic mixture, pharmaceutically acceptable salt, hydrate, or solvate thereof, wherein the method comprises
(i) dissolving a crude compound of formula (IV-F) in dimethyl sulfoxide (DMSO) and ethanol to obtain a solution; and
(ii) conducting crystallization from the solution to obtain a purified compound of formula (IV-F).

The presently claimed method of recrystallizing the compound of formula (IV-F) is particularly suited for industrial scale.

The compounds of formula (IV-F) as well as method of synthesizing the same are disclosed in WO 2021/224489.

R³ is selected from 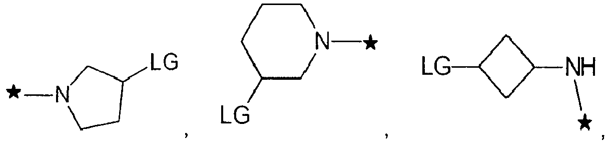 or 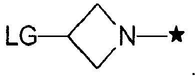 Preferably R³ is selected from 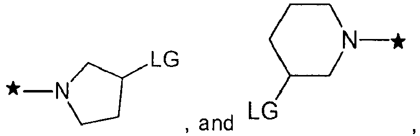 more preferably R³ is

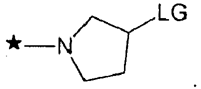

The Leaving Group LG in R³ is C₁₋₄ alkyl sulfonate. Preferably the Leaving Group (LG) is 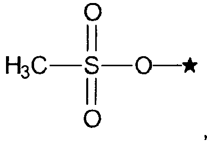 i.e., mesylate.

R⁴ is an aryl, or a 5-membered or 6-membered heteroaryl, wherein R⁴ is selected from 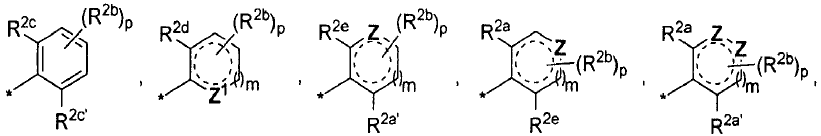 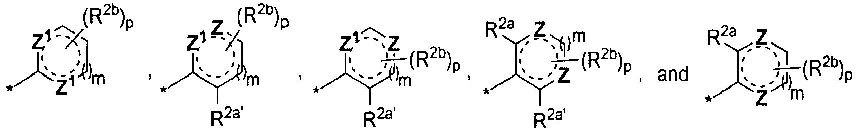 wherein
R^{2a}, R^{2a'} are independently selected from H, or F;
R^{2b} is independently selected from F, -OH, C₁-C₄alkyl, haloC₁-C₄alkyl, -NH₂, -CN, or C₁-C₄alkoxy;
R^{2c}, R^{2c'} are independently selected from H, F, OH, OCH₃, or CH₃;
R^{2d} is selected from H, F, or -OH;
R^{2e} is selected from H, OH, CH₃, or F;
Z is independently N, NH, N(C₁-C₄alkyl), N(haloC₁-C₄alkyl), O, or S;
Z¹ is independently N, NH, O, or S;
p is 0, 1 or 2;
m is 0 or 1;
as valency permits, is a combination of single and double bonds; and
* is the position of bonding.

Preferably **R⁴** is selected from 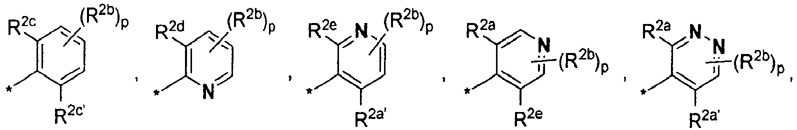 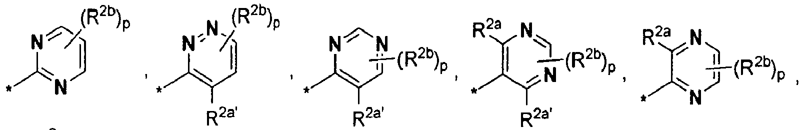 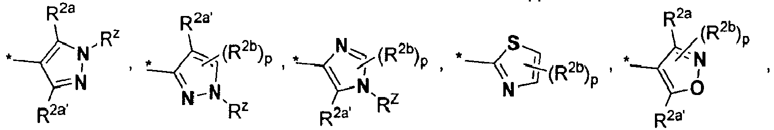 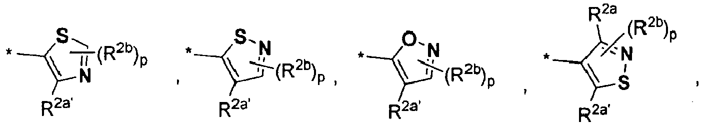 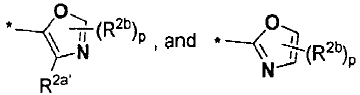 wherein R^{2a}, R^{2a'}, R^{2b}, R^{2c}, R^{2c'}, R^{2d}, R^{2e} and p are as defined hereinabove and R^{z} is selected from H, C₁-C₄alkyl or haloC₁-C₄alkyl.

More preferably, R⁴ is selected from the following: 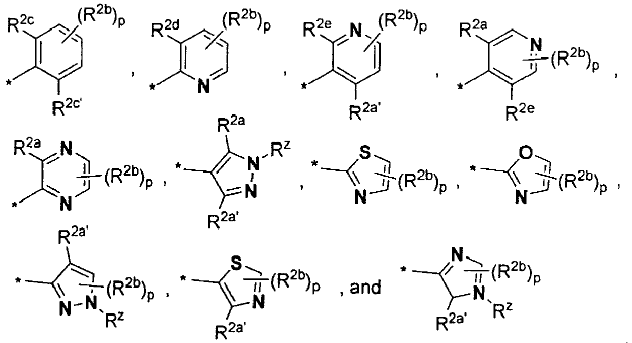 wherein R^{2a}, R^{2a'}, R^{2b}, R^{2c}, R^{2c'}, R^{2d}, R^{2e}, R^{z} and p are as defined hereinabove.

More preferably, R⁴ is selected from the following: 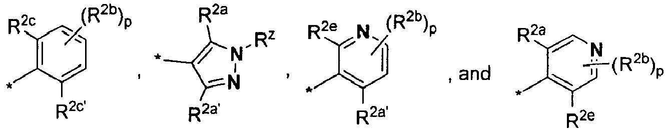 wherein R^{2a}, R^{2a'}, R^{2b}, R^{2c}, R^{2c'}, R^{2e}, R^{z} and p are as defined hereinabove.

Further preferably, **R⁴** is

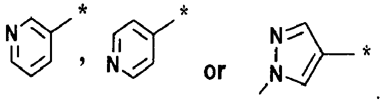

In further preferred embodiment, **R⁴** is

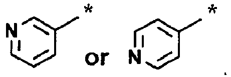

Even more preferably, **R⁴** is

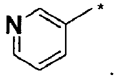

A preferred compound is 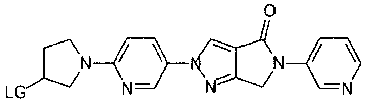 or a pharmaceutically acceptable salt, hydrate, or solvate thereof.

In one embodiment a preferred compound of formula (I) is the following stereoisomer 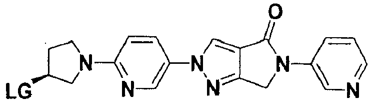 or a pharmaceutically acceptable salt, hydrate, or solvate thereof.

A more preferred compound is 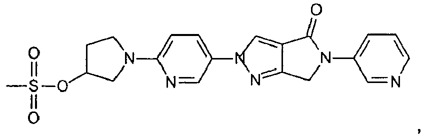 or a pharmaceutically acceptable salt, hydrate, or solvate thereof.

In one embodiment a more preferred compound of formula (I) is the following stereoisomer 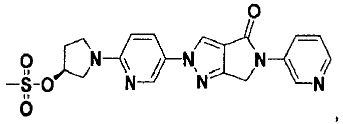 or a pharmaceutically acceptable salt, hydrate, or solvate thereof.

The crude compound of formula (IV-F) which is employed in the method of the present invention can be prepared by the methods set out in WO 2021/224489. In one embodiment, the crude compound of formula (IV-F) can be prepared as shown in the following schemes:

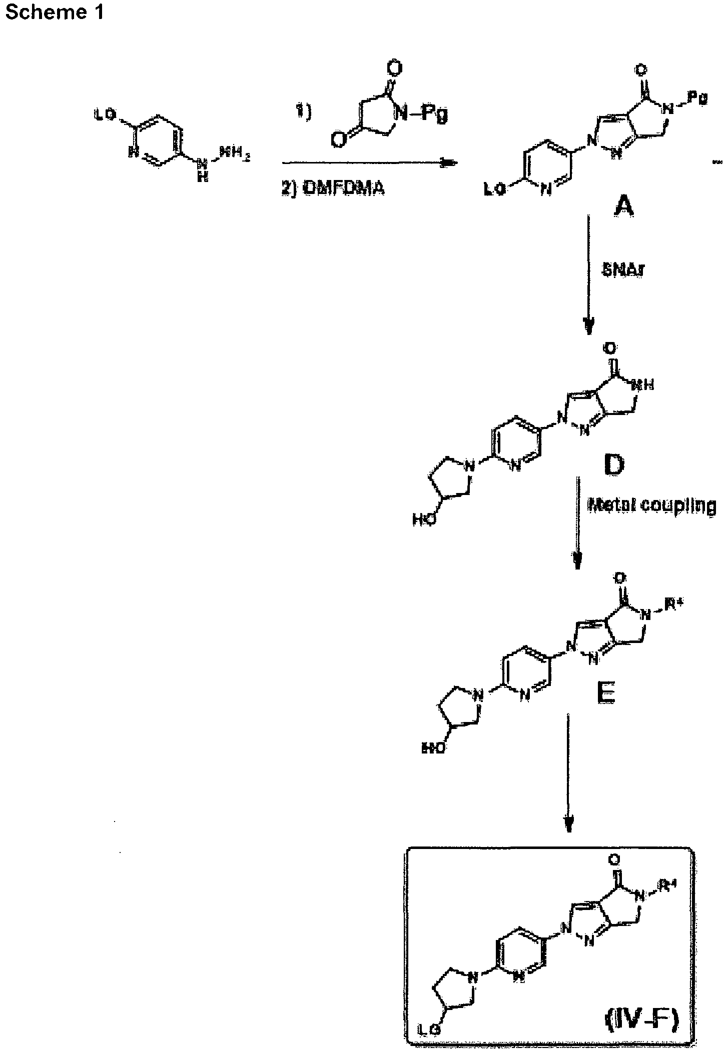

Pg is a protecting group and LG is a leaving group.

Commercially available hydrazine can be condensed with the appropriate ketone to afford the corresponding hydrazone. The crude hydrazone can be subjected to ring cyclization using DMF/DMA to give intermediate A. SNAr can be conducted with a suitable nucleophile in a suitable solvent to give intermediate D.

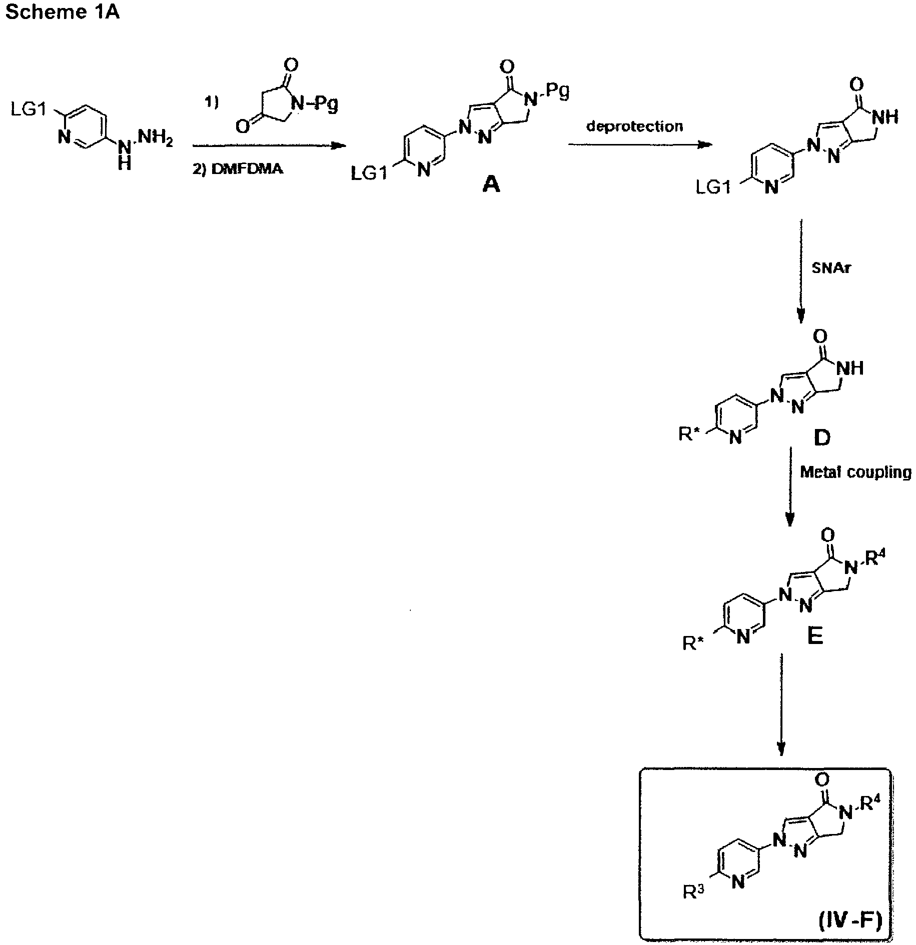

An alternative approach (Scheme 1A) comprises deprotecting intermediate A, followed by SNAr reaction with a suitable nucleophile which is preferably conducted in the presence of CsF in DMSO. Intermediate D can be further functionalized, preferably using copper (I) (Ullmann reaction) in the presence of a base and solvent to afford intermediate E. Finally, LG can be introduced into intermediate E to give a crude compound of formula (IV-F).

In a first reaction step, 2-LG1-5-hydrazinylpyridine can be reacted with 2,4-dioxopyrrolidine which has been suitably protected (e.g., tert-butyl 2,4-dioxopyrrolidine-1-carboxylate). The leaving group LG1 is not specifically limited and can be any leaving group which can be replaced in the subsequent SNAr reaction. Examples thereof include Hal (such as Cl, Br, I, F). The reaction can, for instance, be conducted at elevated temperatures in an appropriate solvent such as a C₁₋₄ alcohol (preferably ethanol). Ring closure of the resultant hydrazone can be conducted as appropriate, for instance, using DMF-DMA, resulting in intermediate A.

The protecting group Pg can be removed using suitable conditions which will depend on the protecting group chosen. Acid cleavage can be given as an example.

A group R* which is a precursor of the desired group R³ can be introduced via an SNAr reaction (nucleophilic aromatic substitution reaction) which results in Intermediate D. This type of reaction is well-known in chemistry and can be conducted under any suitable conditions. Appropriate catalysts such as cesium fluoride can be used, if desired.

R* can be selected from 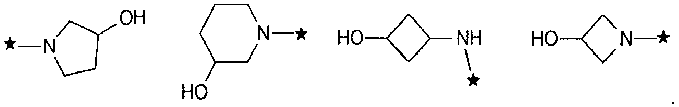 or correspondingly moieties in which the OH is protected by a protecting group.

The group R⁴ can be introduced, for instance, by coupling Intermediate D with R⁴-Hal or R⁴-triflate, resulting in compound E. Coupling reagents include Cu-, Pd- and Ni-containing compounds such as Cul.

In a final step, the crude compound of formula (IV-F) can be prepared by replacing the OH group of Intermediate E by the C₁₋₄ alkyl sulfonate leaving group LG. This reaction can, for instance, be conducted by reacting the Intermediate E with C₁₋₄ alkyl sulfonate-Hal (wherein Hal is, e.g., Cl, such as mesyl chloride).

The above reaction scheme is advantageous because it allows the compound of formula (IV-F) to be prepared in gram amounts rather than in milligram amounts. Furthermore, the crude compound of formula (IV-F) which is obtained by this method has an initial purity of typically more than 90 %. Thus, it is an efficient starting material for a GMP grade purification process.

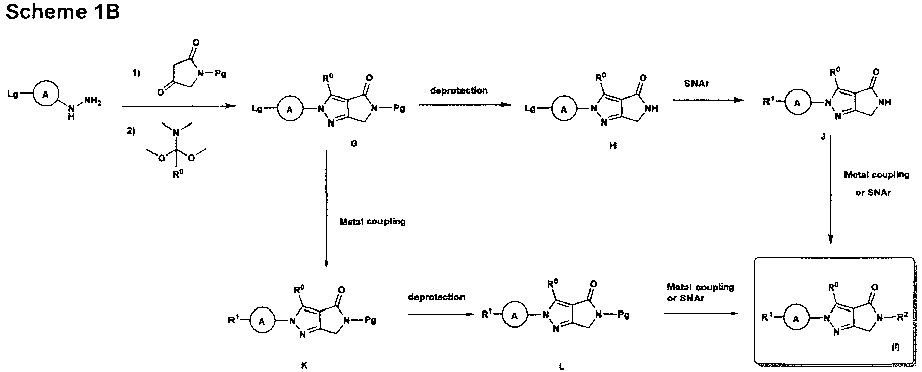

A further general approach is depicted in scheme 1B following the same preferred conditions as described in the general scheme 1 or 1A.

In scheme 1B Lg is a leaving group, Pg is a protecting group, 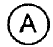 is 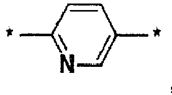 , R¹ is R³, R² is R⁴ and R⁰ is H.

Alternatively, a ¹⁸F-precursor can be obtained by treating intermediate A with hydroxypyrrolidine under heating in a suitable solvent. The R⁴ group can be introduced by palladium catalyzed amidation or Ullmann reaction. Ultimately, an alcohol intermediate E can be modified into a leaving group using standard conditions to give a crude compound of formula (IV-F).

### Step (i)

In step (i), the crude compound of formula (IV-F) is dissolved in a solvent comprising (preferably consisting of) DMSO and ethanol. The ratio (v:v) of DMSO to ethanol is not particularly limited.

The ratio of DMSO to ethanol is typically in the range of about 5:1 to about 0.1:1, preferably about 4:1 to about 0.5:1, more preferably about 3:1 to about 0.5:1, even more preferably about 2:1 to about 0.75:1, most preferably about 1:1. In this context, the ratio (v:v) means a ratio of a unit volume of DMSO to a unit volume of ethanol, e.g., in ml : ml.

The ratio of the solvent to the crude compound of formula (IV-F) is not particularly limited as long as the amount of solvent is sufficient to dissolve the crude compound of formula (IV-F). If the amount of solvent is very large the costs are increased and the crystallization takes a long time. Therefore, it is preferred that the ratio of the solvent to the crude compound of formula (IV-F) (v:w) is in the range of about 5:1 to about 500:1, preferably about 25:1 to about 300:1, more preferably about-40:1 to about 250:1. It is understood that this ratio can vary depending, e.g., on the conditions which are employed during the dissolution step, e.g., the temperature chosen. In this context, the ratio (v:w) means a ratio of a unit volume of the total solvent to a unit weight of crude compound of formula (IV-F), e.g., in ml : g.

The crude compound of formula (IV-F) can be dissolved in the solvent in any suitable manner.

In one embodiment, ethanol and DMSO can be mixed and then the crude compound of formula (IV-F) can be added. In a further embodiment, the crude compound of formula (IV-F) can be mixed with ethanol and then DMSO can be added. In a further preferred embodiment, the crude compound of formula (IV-F) can be mixed with DMSO and then ethanol can be added.

If desired, the dissolution can be promoted by employing elevated temperatures such as a temperature in the range of about 50 to about 90 °C, preferably about 60 to about 90 °C, more preferably about 65 to about 85 °C. These temperatures are given for ambient pressure (approx. 1 atm). If an elevated or reduced pressure is applied, the temperature ranges can be adapted accordingly.

The major amount of the crude compound of formula (IV-F) should be dissolved during the dissolution step (i). Typically, at least about 50 wt.%, preferably at least about 60 wt.%, even more preferably at least about 70 wt.%, further preferably at least about 80 wt.-%, even further preferably at least about 90 wt.%, most preferably 100 % are dissolved.

Conventional measures such as stirring can be employed during the dissolution step (i) in order to enhance or accelerate the dissolution.

### Step (ii)

After the dissolution step (i), the compound of formula (IV-F) is crystallized from the solution to obtain a purified compound of formula (IV-F).

Crystallization can be conducted by various measures such as cooling the solution, removing the solvent or the like. The solvent can be removed, for instance, by reducing the pressure or by heating the solution, so that the solvent evaporates.

In a preferred embodiment, the solution is cooled in order to crystallize the compound of formula (IV-F). The temperature can be chosen appropriately and can, for instance, be in the range of about 0 °C to about 50 °C, preferably about 0 °C to about 40 °C, more preferably about 0 °C to about 30 °C. If desired, the temperature can be varied within this range during the crystallization step. For instance, the crystallization can be initially conducted at about 10 °C to about 50 °C (preferably about 0 °C to about 40 °C, more preferably about 0 °C to about 30 °C) and then the temperature can be reduced to about 0 °C to about 10 °C to improve the yield.

### Optional step (iii)

Finally, at least part (typically all) of the crystallization solution can be removed, so that a purified compound of formula (IV-F) is obtained. Filtration, centrifugation and the like can be employed.

If desired, the obtained crystals can be washed, e.g., with a solvent such as ethanol, and dried in step (iii).

### Further steps

Step (i), step (ii) and optionally step (iii) can be conducted one or more times (e.g., at least 1 time, preferably at least 2 times, more preferably at least 3 times, even more preferably at least 4 times, further preferably at least 5 times) by using the purified compound of formula (IV-F) as a crude compound of formula (IV-F) in step (i) in order to further increase the purity to the desired level.

In a preferred embodiment step (i), step (ii) and optionally step (iii) are conducted at least twice, preferably at least three times.

In another preferred embodiment step (i), step (ii) and optionally step (iii) are conducted at least four times. In a further preferred embodiment step (i), step (ii) and optionally step (iii) are conducted at least five times.

In one embodiment step (i), step (ii) and optionally step (iii) are conducted twice. In another embodiment step (i), step (ii) and optionally step (iii) are conducted three times. In one preferred embodiment step (i), step (ii) and optionally step (iii) are conducted four times. In another preferred embodiment step (i), step (ii) and optionally step (iii) are conducted five times.

In another embodiment, steps (i), step (ii) and optional step (iii) can be conducted at least 6 times, at least 7 times, at least 8 times, at least 9 times, or at least 10 times.

The maximum number of repetitions is not particularly limited. The method of the invention can be repeated as often as desired until the required purity is achieved. However, since the overall yield is reduced when the method of the invention is repeated, step (i), step (ii) and optional step (iii) will be typically conducted at most 30 times, preferably at most 25 times, more preferably at most 20 times, even more preferably at most 15 times.

### Optional purification steps

The method of the present invention can be combined with other purification steps, conventional in the art, as desired. Examples of further purification steps include recrystallization using other solvent systems or chromatography.

In one embodiment, a solvent system using DMSO and 2-butanone or ethyl acetate can be used to dissolve the compound of formula (IV-F). The compound of formula (IV-F) can then be crystallized and then at least part of the solution can be removed, followed by optional drying and/or washing.

The purification using DMSO and 2-butanone or ethyl acetate can be conducted at any point of time but is typically conducted before the method of the present invention or after the method of the present invention, more typically after the method of the present invention. It can be conducted after the complete sequence of step (i), step (ii) and optional step (iii) including the optional repetitions thereof has been conducted or it can be conducted after one or more sequences of step (i), step (ii) and optional step (iii) but before a repetition of step (i), step (ii) and optional step (iii) takes place. In a preferred embodiment, the purification using DMSO and 2-butanone or ethyl acetate is employed after the complete sequence of step (i), step (ii) and optional step (iii) including the optional repetitions thereof has been conducted (e.g., after step (i), step (ii) and optional step (iii) have been conducted at least twice, preferably at least 3 times) because this sequence of steps improves the purity compared to conducting the purification using DMSO and 2-butanone or ethyl acetate at an earlier stage.

The ratio (v:v) of DMSO to 2-butanone is not particularly limited. The ratio of DMSO to 2-butanone is typically in the range of about 5:1 to about 0.1:1, preferably about 4:1 to about 0.5:1, more preferably about 3:1 to about 0.5:1, even more preferably about 2:1 to about 0.75:1, most preferably about 1:1. In this context, the ratio (v:v) means a ratio of a unit volume of DMSO to a unit volume of 2-butanone, e.g., in ml : ml.

The ratio (v:v) of DMSO to ethyl acetate is not particularly limited. The ratio of DMSO to ethyl acetate is typically in the range of about 5:1 to about 0.1:1, preferably about 4:1 to about 0.5:1, more preferably about 3:1 to about 0.5:1, even more preferably about 2:1 to about 0.75:1, most preferably about 1:1. In this context, the ratio (v:v) means a ratio of a unit volume of DMSO to a unit volume of ethyl acetate, e.g., in ml : ml.

The ratio of the solvent to the crude compound of formula (IV-F) is not particularly limited as long as the amount of solvent is sufficient to dissolve the crude compound of formula (IV-F). If the amount of solvent is very large the costs are increased and the crystallization takes a long time. Therefore, it is preferred that the ratio of the solvent to the crude compound of formula (IV-F) (v:w) is in the range of about 5:1 to about 500:1, preferably about 25:1 to about 300:1, more preferably about 40:1 to about 250:1. It is understood that this ratio can vary depending, e.g., on the conditions which are employed during the dissolution step, e.g., the temperature chosen. In this context, the ratio (v:w) means a ratio of a unit volume of the total solvent to a unit weight of crude compound of formula (IV-F), e.g., in ml : g.

The crude compound of formula (IV-F) can be dissolved in the solvent in any suitable manner.

In one embodiment, 2-butanone or ethyl acetate and DMSO can be mixed and then the crude compound of formula (IV-F) can be added. In a further embodiment, the crude compound of formula (IV-F) can be suspended in 2-butanone or ethyl acetate and then DMSO can be added. In a further preferred embodiment, the crude compound of formula (IV-F) can be suspended in DMSO and then 2-butanone or ethyl acetate can be added

If desired, the dissolution can be promoted by employing elevated temperatures such as a temperature in the range of about 50 to about 90 °C, preferably about 60 to about 90 °C, more preferably about 65 to about 85 °C. These temperatures are given for ambient pressure (approx. 1 atm). If an elevated or reduced pressure is applied, the temperature ranges can be adapted accordingly.

The major amount of the crude compound of formula (IV-F) should be dissolved during the dissolution step (i). Typically, at least about 50 wt.%, preferably at least about 60 wt.%, even more preferably at least about 70 wt.%, further preferably at least about 80 wt.-%, even further preferably at least about 90 wt.%, most preferably 100 % are dissolved.

Conventional measures such as stirring can be employed during the dissolution step in order to enhance or accelerate the dissolution.

After the dissolution step, the compound of formula (IV-F) is crystallized from the solution to obtain a purified compound of formula (IV-F).

Crystallization can be conducted by various measures such as cooling the solution, removing the solvent or the like. The solvent can be removed, for instance, by reducing the pressure or by heating the solution, so that the solvent evaporates.

In a preferred embodiment, the solution is cooled in order to crystallize the purified compound of formula (IV-F). The temperature can be chosen appropriately and can, for instance, be in the range of about 0 °C to about 50 °C, preferably about 0 °C to about 40 °C, more preferably about 0 °C to about 30 °C. For instance, the crystallization can be initially conducted at about 10 °C to about 50 °C (preferably about 0 °C to about 40 °C, more preferably about 0 °C to about 30 °C) and then the temperature can be reduced to about 0 °C to about 10 °C to improve the yield. Typically, the purified compound of formula (IV-F) is isolated from the crystallization solution. Filtration, centrifugation and the like can be employed.

If desired, the obtained crystals can be washed, e.g., with a solvent such as 2-butanone or ethyl acetate. The crystals can also be dried, if required.

The desired final purity will depend on the intended use. For diagnostic applications purities of at least 97 % are typically required (Ph. Eur. 2902 "Chemical Precursors for Radiopharmaceutical Preparations"). The conditions during step (i), step (ii) and optional step (iii) can be the same or different during the repetitions.

It has been surprisingly found that the purified compound of formula (IV-F) which has been prepared by the method of the present invention not only has a higher purity than the crude compound of formula (IV-F) but also has an improved stability. For instance, the purified compound of formula (IV-F) which had been subjected to three recrystallizations was stable under accelerated storage conditions (40 °C for 4 weeks). In contrast thereto, the crude compound of formula (IV-F) degraded by 15.2 wt.% under these conditions.

The advantages of the present invention are observed with compounds in which LG is C₁₋₄ alkyl sulfonate, particularly mesylate.

### METHOD OF SYNTHESIS OF DETECTABLY LABELLED COMPOUNDS

The compounds which are obtainable by the method of the present invention are suitable as a precursor of a detectably labelled compound which can be used in imaging applications.

In one embodiment, the present invention relates to a method for preparing a compound of formula (III-F), or a stereoisomer, racemic mixture, pharmaceutically acceptable salt, hydrate, or solvate thereof, by radiolabeling a compound of formula (IV-F) with the radioisotope ¹⁸F.

The reagents, solvents and conditions which can be used for the ¹⁸F-fluorination are well-known to a skilled person in the field (L. Cai, S. Lu, V. Pike, Eur. J. Org. Chem 2008, 2853-2873; J. Fluorine Chem., 27 (1985):177-191; Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). Preferably, the solvents used in the ¹⁸F-fluorination are DMF, DMSO, acetonitrile, DMA, or mixtures thereof, preferably the solvent is acetonitrile or DMSO.

Any suitable ¹⁸F-fluorinating agent can be employed. Typical examples include H¹⁸F, alkali or alkaline earth ¹⁸F-fluorides (e.g., K¹⁸F, Rb¹⁸F, Cs¹⁸F, and Na¹⁸F). Optionally, the ¹⁸F-fluorination agent can be used in combination with a chelating agent such as a cryptand (e.g.: 4,7,13,16,21,24-hexaoxa-1,10-diazabicyclo[8.8.8]-hexacosane - Kryptofix^{®}) or a crown ether (e.g.: 18-crown-6). Alternatively, the ¹⁸F-fluorinating agent can be a tetraalkylammonium salt of ¹⁸F or a tetraalkylphosphonium salt of ¹⁸F; e.g., tetra(C₁₋₆ alkyl)ammonium salt of ¹⁸F or a tetra(C₁₋₆ alkyl)phosphonium salt of ¹⁸F. Preferably, the ¹⁸F-fluorination agent is K¹⁸F, H¹⁸F, Cs¹⁸F, Na¹⁸F tetra(C₁₋₆ alkyl) ammonium salt of ¹⁸F, kryptofix[222]¹⁸F or tetrabutylammonium [¹⁸F]fluoride.

### DIAGNOSTIC COMPOSITIONS

The compounds of formula (III-F), or a stereoisomer, racemic mixture, pharmaceutically acceptable salt, hydrate, or solvate thereof, are particularly suitable for imaging of alpha-synuclein aggregates. With respect to alpha-synuclein protein, the compounds are particularly suitable for binding to various types of alpha-synuclein aggregates. The imaging can be conducted in mammals, preferably in humans. The imaging is preferably *in vitro* imaging, *ex vivo* imaging, or *in vivo* imaging. More preferably the imaging is *in vivo* imaging: Even more preferably, the imaging is preferably brain imaging. The imaging can also be eye/retinal imaging. The compounds of formula (III-F), or a stereoisomer, racemic mixture, pharmaceutically acceptable salt, hydrate, or solvate thereof, are particularly suitable for use in diagnostics.

The diagnostics can be conducted for mammals, preferably for humans. The tissue of interest on which the diagnostics is conducted can be brain, tissue of the central nervous system, tissue of the eye (such as retinal tissue) or other tissues, peripheral organs such as, but not limited to, the gut, or body fluids such as cerebrospinal fluid (CSF). The tissue is preferably brain tissue.

Due to their design and to the binding characteristics, the compounds of formula (III-F), or a stereoisomer, racemic mixture, pharmaceutically acceptable salt, hydrate, or solvate thereof, are suitable for use in the diagnosis of diseases, disorders and abnormalities associated with alpha-synuclein aggregates. The compounds of formula (III-F), or a stereoisomer, racemic mixture, pharmaceutically acceptable salt, hydrate, or solvate thereof, are particularly suitable for positron emission tomography imaging of alpha-synuclein aggregates. Diseases involving alpha-synuclein aggregates are generally listed as synucleinopathies (or α-synucleinopathies). The compounds of formula (III-F), or a stereoisomer, racemic mixture, pharmaceutically acceptable salt, hydrate, or solvate thereof, are suitable for use in the diagnosis of diseases, disorders or abnormalities including, but not limited to, Parkinson's disease (sporadic, familial with alpha-synuclein mutations, familial with mutations other than alpha-synuclein, pure autonomic failure and Lewy body dysphagia), SNCA duplication carrier, dementia with Lewy bodies ("pure" Lewy body dementia), Alzheimer's disease, sporadic Alzheimer's disease, familial Alzheimer's disease with APP mutations, familial Alzheimer's disease with PS-1, PS-2 or other mutations, familial British dementia, Lewy body variant of Alzheimer's disease and normal aging in Down syndrome). Synucleinopathies with neuronal and glial aggregates of alpha synuclein include multiple system atrophy (MSA) (Shy-Drager syndrome, striatonigral degeneration and olivopontocerebellar atrophy). Other diseases that may have alpha-synuclein-immunoreactive lesions include traumatic brain injury, chronic traumatic encephalopathy, tauopathies (Pick's disease, frontotemporal dementia, progressive supranuclear palsy, corticobasal degeneration and Niemann-Pick type C1 disease), motor neuron disease, amyotrophic lateral sclerosis (sporadic, familial and ALS-dementia complex of Guam), neuroaxonal dystrophy, neurodegeneration with brain iron accumulation type 1 (Hallervorden-Spatz syndrome), prion diseases, ataxia telangiectatica, Meige's syndrome, subacute sclerosing panencephalitis, Gaucher disease as well as other lysosomal storage disorders (including Kufor-Rakeb syndrome and Sanfilippo syndrome) and rapid eye movement (REM) sleep behavior disorder (Jellinger, Mov Disord 2003, 18 Suppl. 6, S2-12; Galvin et al. JAMA Neurology 2001, 58 (2), 186-190; Kovari et al., Acta Neuropathol. 2007, 114(3), 295-8; Saito et al., J Neuropathol Exp Neurol. 2004, 63(4), 323-328; McKee et al., Brain, 2013, 136(Pt 1), 43-64; Puschmann et al., Parkinsonism Relat Disord 2012, 18S1, S24-S27; Usenovic et al., J Neurosci. 2012, 32(12), 4240-4246; Winder-Rhodes et al., Mov Disord. 2012, 27(2), 312-315; Ferman et al., J Int Neuropsychol Soc. 2002, 8(7), 907-914). Preferably, the compounds of formula (III-F) are suitable for use in the diagnosis of Parkinson's disease, multiple system atrophy, dementia with Lewy bodies, Parkinson's disease dementia, SNCA duplication carrier, or Alzheimer's disease, more preferably multiple system atrophy (MSA).

In the methods of diagnosing a disease, disorder or abnormality associated with alpha-synuclein aggregates, such as multiple system atrophy (MSA), or a predisposition therefor in a subject, the method comprises the steps of:
a) administering to the subject a diagnostically effective amount of a compound of formula (III-F), or a stereoisomer, racemic mixture, pharmaceutically acceptable salt, hydrate, or solvate thereof;
b) allowing the compound of formula (III-F), or a stereoisomer, racemic mixture, pharmaceutically acceptable salt, hydrate, or solvate thereof, to distribute into the tissue of interest (such as brain, tissue of the central nervous system (CNS), eye tissue or other tissues, peripheral organs such as, but not limited to, the gut, or body fluids such as cerebrospinal fluid (CSF)); and
c) imaging the tissue of interest, wherein an increase in binding of the compound of formula (III-F), or a stereoisomer, racemic mixture, pharmaceutically acceptable salt, hydrate, or solvate thereof, to the tissue of interest compared to a normal control level of binding indicates that the subject is suffering from or is at risk of developing a disease, disorder or abnormality associated with alpha-synuclein aggregates.

The compounds of formula (III-F), or a stereoisomer, racemic mixture, pharmaceutically acceptable salt, hydrate, or solvate thereof, can be used for imaging of alpha-synuclein aggregates in any sample or a specific body part or body area of a patient which is suspected to contain alpha-synuclein aggregates. The compounds are able to pass the blood-brain barrier. Consequently, they are particularly suitable for imaging of alpha-synuclein aggregates in the brain, tissue of the central nervous system (CNS), or eye tissue or in peripheral organs such as, but not limited to, the gut, as well as in body fluids such as cerebrospinal fluid (CSF).

In diagnostic applications, the compounds of formula (III-F), or a stereoisomer, racemic mixture, pharmaceutically acceptable salt, hydrate, or solvate thereof, are preferably administered in the form of a diagnostic composition comprising the compound of formula (III-F), or a stereoisomer, racemic mixture, pharmaceutically acceptable salt, hydrate, or solvate thereof. A "diagnostic composition" is defined in the present invention as a composition comprising one or more compounds of formula (III-F), or a stereoisomer, racemic mixture, pharmaceutically acceptable salt, hydrate, or solvate thereof, in a form suitable for administration to a patient, e.g., a mammal such as a human, and which is suitable for use in the diagnosis of the specific disease, disorder or abnormality at issue. Preferably a diagnostic composition further comprises a physiologically acceptable excipient, carrier, diluent or adjuvant. Administration is preferably carried out as defined below. More preferably by injection of the composition as an aqueous solution. Such a composition may optionally contain further ingredients such as buffers; pharmaceutically acceptable solubilisers (e.g., cyclodextrins or surfactants such as Pluronic, Tween or phospholipids); and pharmaceutically acceptable stabilisers or antioxidants (such as ascorbic acid, gentisic acid or para-aminobenzoic acid). The dose of the compound of formula (III-F), or a stereoisomer, racemic mixture, pharmaceutically acceptable salt, hydrate, or solvate thereof, will vary depending on the exact compound to be administered, the weight of the patient, and other variables as would be apparent to a physician skilled in the art.

While it is possible for the compounds of formula (III-F), or a stereoisomer, racemic mixture, pharmaceutically acceptable salt, hydrate, or solvate thereof, to be administered alone, it is preferable to formulate them into a diagnostic composition in accordance with standard pharmaceutical practice. Thus, the invention also provides a diagnostic composition which comprises a diagnostically effective amount of a compound of formula (III-F), or a stereoisomer, racemic mixture, pharmaceutically acceptable salt, hydrate, or solvate thereof, in admixture with, optionally, at least one pharmaceutically acceptable excipient, carrier, diluent or adjuvant.

Pharmaceutically acceptable excipients are well known in the pharmaceutical art, and are described, for example, in Remington's Pharmaceutical Sciences, 15th Ed., Mack Publishing Co., New Jersey (1975). The pharmaceutical excipient can be selected with regard to the intended route of administration and standard pharmaceutical practice. The excipient must be acceptable in the sense of being not deleterious to the recipient thereof.

Pharmaceutically useful excipients, carriers, adjuvants and diluents that may be used in the formulation of the diagnostic composition of the present invention may comprise, for example, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate, binders, adjuvants, solubilizers, thickening agents, stabilizers, disintegrants, glidants, lubricating agents, buffering agents, emulsifiers, wetting agents, suspending agents, sweetening agents, colorants, flavors, coating agents, preservatives, antioxidants, processing agents, drug delivery modifiers and enhancers such as calcium phosphate, magnesium stearate, talc, monosaccharides, disaccharides, starch, gelatin, cellulose, methylcellulose, sodium carboxymethyl cellulose, dextrose, hydroxypropyl-β-cyclodextrin, polyvinylpyrrolidone, low melting waxes, and ion exchange resins.

The routes for administration (delivery) of the compounds of formula (III-F), or a stereoisomer, racemic mixture, pharmaceutically acceptable salt, hydrate, or solvate thereof are not limited but are preferably via injections, more preferably via intravenous injection.

Preferably, in diagnostic applications, the compounds of formula (III-F), or a stereoisomer, racemic mixture, pharmaceutically acceptable salt, hydrate, or solvate thereof, are administered parenterally. If the compounds of formula (III-F), or a stereoisomer, racemic mixture, pharmaceutically acceptable salt, hydrate, or solvate thereof, are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds; and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing diagnosis.

The diagnostic compositions of the invention can be produced in a manner known per se to the skilled person as described, for example, in Remington's Pharmaceutical Sciences, 15th Ed., Mack Publishing Co., New Jersey (1975).

The compounds of formula (III-F), or a stereoisomer, racemic mixture, pharmaceutically acceptable salt, hydrate, or solvate thereof, are useful as an *in vitro* analytical reference or an *in vitro* screening tool. They are also useful in *in vivo* diagnostic methods.

The compounds of formula (III-F), or a stereoisomer, racemic mixture, pharmaceutically acceptable salt, hydrate, or solvate thereof, can also be provided in the form of a mixture comprising a compounds of formula (III-F), or a stereoisomer, racemic mixture, pharmaceutically acceptable salt, hydrate, or solvate thereof, and at least one compound selected from an imaging agent different from the compound of formula (III-F), a pharmaceutically acceptable excipient, carrier, diluent or adjuvant. The imaging agent different from the compound of formula (III-F) is preferably present in a diagnostically effective amount. More preferably the imaging agent different from the compound of formula (III-F) is an amyloid beta or Tau imaging agent.
- Diagnosis of a disease, disorder or abnormality associated with alpha-synuclein aggregates or of a predisposition to a disease, disorder or abnormality associated with alpha-synuclein aggregates in a patient may be achieved by detecting the specific binding of a compound of formula (III-F), or a stereoisomer, racemic mixture, pharmaceutically acceptable salt, hydrate, or solvate thereof, to the alpha-synuclein aggregates in a sample or a specific body part or body area, which includes the steps of:
   (a) bringing the sample or a specific body part or body area suspected to contain the alpha-synuclein aggregates into contact with a compound of formula (III-F), or a stereoisomer, racemic mixture, pharmaceutically acceptable salt, hydrate, or solvate thereof, which binds the alpha-synuclein aggregates,
   (b) allowing the compound of formula (III-F), or a stereoisomer, racemic mixture, pharmaceutically acceptable salt, hydrate, or solvate thereof, to bind to the alpha-synuclein aggregates to form a compound/(alpha-synuclein aggregates) complex (hereinafter "compound/(alpha-synuclein aggregates) complex" will be abbreviated as "compound/protein aggregate complex"),
   (c) detecting the formation of the compound/protein aggregate complex,
   (d) optionally correlating the presence or absence of the compound/protein aggregate complex with the presence or absence of alpha-synuclein aggregates in the sample or specific body part or area, and
   (e) optionally comparing the amount of the compound/protein aggregate complex to a normal control value, wherein an increase in the amount of the compound/protein aggregate complex compared to a normal control value may indicate that the patient is suffering from or is at risk of developing a disease, disorder or abnormality associated with alpha-synuclein aggregates.

The compound of formula (III-F), or a stereoisomer, racemic mixture, pharmaceutically acceptable salt, hydrate, or solvate thereof, can be brought into contact with the sample or the specific body part or body area suspected to contain the alpha-synuclein aggregates by a suitable method. In *in vitro* methods the compound of formula (III-F), or a stereoisomer, racemic mixture, pharmaceutically acceptable salt, hydrate, or solvate thereof, and a liquid sample can be simply mixed. In *in vivo* tests the compound of formula (III-F), or a stereoisomer, racemic mixture, pharmaceutically acceptable salt, hydrate, or solvate thereof, is typically administered to the patient by any suitable means such as parenteral administration, with intravenous injection being preferred.

After the sample or a specific body part or body area has been brought into contact with the compound of formula (III-F), or a stereoisomer, racemic mixture, pharmaceutically acceptable salt, hydrate, or solvate thereof, the compound is allowed to bind to the alpha-synuclein aggregates. The amount of time required for binding will depend on the type of test (e.g., *in vitro* or *in vivo)* and can be determined by a person skilled in the field by routine experiments.

The compound which has bound to the alpha-synuclein aggregates, can be subsequently detected by any appropriate method. The specific method chosen will depend on the detectable label which has been chosen. Examples of possible methods include, but are not limited to, a fluorescence imaging technique or a nuclear imaging technique such as positron emission tomography (PET), single photon emission computed tomography (SPECT), magnetic resonance imaging (MRI), and contrast-enhanced magnetic resonance imaging (MRI). The fluorescence imaging technique and/or nuclear imaging technique can be employed for monitoring and/or visualizing the distribution of the detectably labelled compound within the sample or a specific body part or body area.

The presence or absence of the compound/protein aggregate complex is then optionally correlated with the presence or absence of alpha-synuclein aggregates in the sample or specific body part or area. Finally, the amount of the compound/protein aggregate complex can be compared to a normal control value which has been determined in a sample or a specific body part or body area of a healthy subject, wherein an increase in the amount of the compound/protein aggregate complex compared to a normal control value may indicate that the patient is suffering from or is at risk of developing a disease, disorder or abnormality associated with alpha-synuclein aggregates.

The present invention also relates to a method of determining the amount of alpha-synuclein aggregates in a tissue and/or a body fluid. This method comprises the steps of:
(a) providing a sample representative of the tissue and/or body fluid under investigation;
(b) testing the sample for the presence of alpha-synuclein aggregates with a compound of formula (III-F);
(c) determining the amount of compound bound to the alpha-synuclein aggregates; and
(d) calculating the amount of alpha-synuclein aggregates in the tissue and/or body fluid.

The sample can be tested for the presence of alpha-synuclein aggregates with a compound of formula (III-F), or a stereoisomer, racemic mixture, pharmaceutically acceptable salt, hydrate, or solvate thereof, by bringing the sample into contact with a compound of formula (III-F), allowing the compound of formula (III-F) to bind to the alpha-synuclein aggregates to form a compound/protein aggregate complex and detecting the formation of the compound/protein aggregate complex as explained above.

Monitoring minimal residual disease, disorder or abnormality in a patient suffering from a disease, disorder or abnormality associated with alpha-synuclein aggregates who has been treated with a medicament with a compound of formula (III-F), or a stereoisomer, racemic mixture, pharmaceutically acceptable salt, hydrate, or solvate thereof, may be achieved by
(a) bringing a sample or a specific body part or body area suspected to contain alpha-synuclein aggregates into contact with a compound of formula (III-F), or a stereoisomer, racemic mixture, pharmaceutically acceptable salt, hydrate, or solvate thereof;
(b) allowing the compound to bind to the alpha-synuclein aggregates to form a compound/protein aggregate complex;
(c) detecting the formation of the compound/protein aggregate complex;
(d) optionally correlating the presence or absence of the compound/protein aggregate complex with the presence or absence of alpha-synuclein aggregates in the sample or specific body part or body area; and
(e) optionally comparing the amount of the compound/protein aggregate complex to a normal control value, wherein an increase in the amount of the aggregate compared to a normal control value may indicate that the patient may still suffer from a minimal residual disease, disorder or abnormality.

How steps (a) to (e) can be conducted has already been explained above.

In the method for monitoring minimal residual disease, disorder or abnormality, the method can further comprises steps (i) to (vi) before step (a):
(i) bringing a sample or specific body part or body area suspected to contain alpha-synuclein aggregates into contact with the compound of formula (III-F), or a stereoisomer, racemic mixture, pharmaceutically acceptable salt, hydrate, or solvate thereof, which compound specifically binds to the alpha-synuclein aggregates;
(ii) allowing the compound to bind to the alpha-synuclein aggregates to form a compound/(alpha-synuclein aggregates) complex;
(iii) detecting the formation of the compound/(alpha-synuclein aggregates) complex;
(iv) correlating the presence or absence of the compound/(alpha-synuclein aggregates) complex with the presence or absence of alpha-synuclein aggregates in the sample or specific body part or body area;
(v) optionally comparing the amount of the compound/(alpha-synuclein aggregates) complex to a normal control value; and
(vi) treating the patient with the medicament.

Optionally the method can further comprise step (A) after step (d) or step (e):
(A) comparing the amount of the compound/(alpha-synuclein aggregates) complex determined in step (iv) to the amount of the compound/(alpha-synuclein aggregates) complex determined in step (d).

In order to monitor minimal residual disease, disorder or abnormality over time, steps (a) to (c) and optionally steps (d) and (e) of the method of monitoring minimal residual disease, disorder or abnormality can be repeated one or more times.

In the method for monitoring minimal residual disease, disorder or abnormality the amount of the compound/protein aggregate complex can be optionally compared at various points of time during the treatment, for instance, before and after onset of the treatment or at various points of time after the onset of the treatment. A change, especially a decrease, in the amount of the compound/protein aggregate complex may indicate that the residual disease, disorder or abnormality is decreasing.

Predicting responsiveness of a patient suffering from a disease, disorder or abnormality associated with alpha-synuclein aggregates and being treated with a medicament can be achieved by
(a) bringing a sample or a specific body part or body area suspected to contain alpha-synuclein aggregates into contact with a compound of formula (III-F), or a stereoisomer, racemic mixture, pharmaceutically acceptable salt, hydrate, or solvate thereof;
(b) allowing the compound to bind to the alpha-synuclein aggregates to form a compound/protein aggregate complex;
(c) detecting the formation of the compound/protein aggregate complex;
(d) optionally correlating the presence or absence of the compound/protein aggregate complex with the presence or absence of alpha-synuclein aggregates in the sample or specific body part or body area; and
(e) optionally comparing the amount of the compound/protein aggregate complex to a normal control value.

How steps (a) to (e) can be conducted has already been explained above.

In the method for predicting the responsiveness, the method can further comprises steps (i) to (vi) before step (a):
(i) bringing a sample or specific body part or body area suspected to contain alpha-synuclein aggregates into contact with the compound of formula (III-F), or a stereoisomer, racemic mixture, pharmaceutically acceptable salt, hydrate, or solvate thereof, which compound specifically binds to the alpha-synuclein aggregates;
(ii) allowing the compound to bind to the alpha-synuclein aggregates to form a compound/(alpha-synuclein aggregates) complex;
(iii) detecting the formation of the compound/(alpha-synuclein aggregates) complex;
(iv) correlating the presence or absence of the compound/(alpha-synuclein aggregates) complex with the presence or absence of alpha-synuclein aggregates in the sample or specific body part or body area;
(v) optionally comparing the amount of the compound/(alpha-synuclein aggregates) complex to a normal control value; and
(vi) treating the patient with the medicament.

Optionally the method can further comprise step (A) after step (d) or step (e):
(A) comparing the amount of the compound/(alpha-synuclein aggregates) complex determined in step (iv) to the amount of the compound/(alpha-synuclein aggregates) complex determined in step (d).

In order to determine the responsiveness over time, steps (a) to (c) and optionally steps (d) and (e) of the method of predicting responsiveness can be repeated one or more times.

In the method for predicting responsiveness the amount of the compound/protein aggregate complex can be optionally compared at various points of time during the treatment, for instance, before and after onset of the treatment or at various points of time after the onset of the treatment. A change, especially a decrease, in the amount of the compound/protein aggregate complex may indicate that the patient has a high potential of being responsive to the respective treatment.

Optionally, the diagnostic composition can be used before, during and after, surgical procedures (e.g. deep brain stimulation (DBS)) and non-invasive brain stimulation (such as repetitive transcranial magnetic stimulation (rTMS)), for visualizing alpha-synuclein aggregates before, during and after such procedures. Surgical techniques, including DBS, improve advanced symptoms of PD on top of the best currently used medical therapy. During the past 2 decades, rTMS has been closely examined as a possible treatment for PD (Ying-hui Chou et al. JAMA Neurol. 2015 April 1; 72(4): 432-440).

In a further embodiment of the invention, the diagnostic composition can be used in a method of collecting data for monitoring residual disease, disorder or abnormality in a patient suffering from a disease, disorder or abnormality associated withlt is understood that the term "monitoring minimal residual disease" as mentioned herein relates to the monitoring of the evolution of the disease. For example, monitoring of the evolution of the disease, disorder or abnormality in a patient suffering from a disease, disorder or abnormality associated with alpha-synuclein aggregates.

A compound of formula (III-F), or a compound of formula (IV-F), or a stereoisomer, racemic mixture, pharmaceutically acceptable salt, hydrate, or solvate thereof, can also be incorporated into a test kit for detecting alpha-synuclein protein aggregates. The test kit typically comprises a container holding one or more compounds of formula (III-F), or one or more compounds of formula (IV-F), or a stereoisomer, racemic mixture, pharmaceutically acceptable salt, hydrate, or solvate thereof, and instructions for using the compound for the purpose of binding to alpha-synuclein aggregates to form a compound/protein aggregate complex and detecting the formation of the compound/protein aggregate complex such that presence or absence of the compound/protein aggregate complex correlates with the presence or absence of the alpha-synuclein aggregates.

The term "test kit" refers in general to any diagnostic kit known in the art. More specifically, the latter term refers to a diagnostic kit as described in Zrein et al., Clin. Diagn. Lab. Immunol., 1998, 5, 45-49.

The dose of the detectably labelled compounds of formula (III-F), or a racemic mixture, pharmaceutically acceptable salt, hydrate, or solvate thereof, will vary depending on the exact compound to be administered, the weight of the patient, size and type of the sample, and other variables as would be apparent to a physician skilled in the art. Generally, the dose could preferably lie in the range 0.001 µg/kg to 10 µg/kg, preferably 0.01 µg/kg to 1.0 µg/kg. The radioactive dose can be, e.g., 100 to 600 MBq, more preferably 150 to 450 MBq.

In another embodiment the present invention provides a method of imaging a disease, disorder or abnormality associated with alpha-synuclein aggregates in a sample or in a a specific body part or body area, in particular in a brain or a sample taken from a patient's brain, the method comprising the steps:
(a) Bringing the sample, the specific body part or body area suspected to contain alpha-synuclein aggregates into contact with a compound of formula (III-F), or a stereoisomer, racemic mixture, pharmaceutically acceptable salt, hydrate, or solvate thereof;
(b) Allowing the compound to bind to the alpha-synuclein aggregates; and
(c) Imaging the sample, the specific body part or the body area with an imaging system.

In another embodiment the present invention provides a method of determining an amount of alpha-synuclein aggregates in a sample or a specific body part or body area, the method comprising the steps:
(a) Bringing the sample, the specific body part or body area suspected to contain alpha-synuclein aggregates into contact with a compound of formula (III-F), or a stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates;
(d) Determining the amount of the compound bound to the alpha-synuclein aggregates; and
(e) Optionally calculating the amount of the alpha-synuclein aggregates in the sample, the specific body part or body area.

In another embodiment the present invention provides a method of imaging a disease, disorder or abnormality associated with alpha-synuclein aggregates, the method comprising the steps:
(a) Bringing a sample, a specific body part or body area suspected to contain alpha-synuclein aggregates into contact with a compound of formula (III-F), or a stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates; and
(d) Correlating the presence or absence of the compound bound to the alpha-synuclein aggregates with the disease, disorder or abnormality associated with the alpha-synuclein aggregates.

In another embodiment the present invention provides a method of collecting data for the diagnosis of a disease, disorder or abnormality associated with alpha-synuclein aggregates, the method comprising the steps:
(a) Bringing a sample or a specific body part or body area suspected to contain alpha-synuclein aggregates into contact with a compound of formula (III-F), or a stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates; and
(d) Optionally correlating the presence or absence of the compound bound to the alpha-synuclein aggregates with the presence or absence of the alpha-synuclein aggregates in the sample or specific body part or body area.

In another embodiment the present invention provides a method of collecting data for determining a predisposition to a disease, disorder or abnormality associated with alpha-synuclein aggregates, the method comprising the steps:
(a) Bringing a sample or a specific body part or body area suspected to contain alpha-synuclein aggregates into contact with a compound of formula (III-F), or a stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates; and
(d) Optionally correlating the presence or absence of the compound bound to the alpha-synuclein aggregates with the presence or absence of the alpha-synuclein aggregates in the sample or specific body part or body area.

If the amount of the compound bound to the alpha-synuclein aggregates is higher than a normal control value of a healthy/reference subject this indicates that the patient is suffering from or is at risk of developing a disease, disorder or abnormality associated with alpha-synuclein aggregates. In particular, if the amount of the compound bound to the alpha-synuclein aggregates is higher than what expected in a person showing no clinical evidence of neurodegenerative disease, it can be assumed that the patient has a disposition to a disease, disorder or abnormality associated with alpha-synuclein aggregates or a synucleinopathy.

In another embodiment the present invention provides a method of collecting data for prognosing a disease, disorder or abnormality associated with alpha-synuclein aggregates, wherein the method comprises the steps:
(a) Bringing a sample, a specific body part or body area suspected to contain alpha-synuclein aggregates into contact with a compound of formula (III-F), or a stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates;
(d) Optionally correlating the presence or absence of the compound bound to the alpha-synuclein aggregates with the presence or absence of the alpha-synuclein aggregates in the sample or specific body part or body area; and
(e) Optionally repeating steps (a) to (c) and, if present, optional step (d) at least one time.

The progression of a disease, disorder or abnormality and/or the prospect (e.g., the probability, duration, and/or extent) of recovery can be estimated by a medical practioner based on the presence or absence of the compound bound to the alpha-synuclein aggregates, the amount of the compound bound to the alpha-synuclein aggregates or the like. If desired, steps (a) to (c) and, if present, optional step (d) can be repeated over time to monitor the progression of the disease, disorder or abnormality and to thus allow a more reliable estimate.

In another embodiment the invention provides a method of collecting data for monitoring the evolution of the disease in a patient suffering from a disease, disorder or abnormality associated with alpha-synuclein aggregates, the method comprising the steps:
(a) Bringing a sample, a specific body part or body area suspected to contain alpha-synuclein aggregates into contact with a compound of formula (III-F), or a detectably labelled compound, stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates;
(d) Optionally correlating the presence or absence of the compound bound to the alpha-synuclein aggregates with the presence or absence of the alpha-synuclein aggregates in the sample or specific body part or body area; and
(e) Optionally repeating steps (a) to (c) and, if present, optional step (d) at least one time.

Typically the patient is or has been undergoing treatment of the disease, disorder or abnormality associated with alpha-synuclein aggregates or is/has been undergoing treatment of the synucleinopathy. In particular, the treatment can involve administration of a medicament which is suitable for treating the disease, disorder or abnormality associated with alpha-synuclein aggregates.

In another embodiment the present invention provides a method of collecting data for monitoring the progression of a disease, disorder or abnormality associated with alpha-synuclein aggregates, in a patient, the method comprising the steps:
(a) Bringing a sample, a specific body part or body area suspected to contain alpha-synuclein aggregates into contact with the compound of formula (III-F), or a stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates;
(d) Optionally correlating the presence or absence of the compound bound to the alpha-synuclein aggregates with the presence or absence of the alpha-synuclein aggregates in the sample or specific body part or body area; and
(e) Optionally repeating steps (a) to (c) and, if present, optional step (d) at least one time.

Typically, the patient is or has been undergoing treatment of the disease, disorder or abnormality associated with alpha-synuclein aggregates or is or has been undergoing treatment of the synucleinopathy. In particular, the treatment can involve administration of a medicament which is suitable for treating the disease, disorder or abnormality associated with alpha-synuclein aggregates.

In another embodiment the invention provides a method of collecting data for predicting responsiveness of a patient suffering from a disease, disorder or abnormality associated with alpha-synuclein aggregates, to a treatment of the disease, disorder or abnormality associated with alpha-synuclein aggregates, the method comprising the steps:
(a) Bringing a sample, a specific body part or body area suspected to contain alpha-synuclein aggregates into contact with a compound of formula (III-F), or a stereoisomer, racemic mixture, pharmaceutically acceptable salt, hydrate, or solvate thereof;
(b) Allowing the compound to bind to the alpha-synuclein aggregates;
(c) Detecting the compound bound to the alpha-synuclein aggregates;
(d) Optionally correlating the presence or absence of the compound bound to the alpha-synuclein aggregates with the presence or absence of the alpha-synuclein aggregates in the sample or specific body part or body area; and
(e) Optionally repeating steps (a) to (c) and, if present, optional step (d) at least one time.

Typically, the patient is or has been undergoing treatment of the disease, disorder or abnormality associated with alpha-synuclein aggregates or is or has been undergoing treatment of the synucleinopathy. In particular, the treatment can involve administration of a medicament which is suitable for treating the disease, disorder or abnormality associated with alpha-synuclein aggregates.

If the amount of the compound bound to the alpha-synuclein aggregates decreases over time, it can be assumed that the patient is responsive to the treatment. If the amount of the compound bound to the alpha-synuclein aggregates is essentially constant or increases over time, it can be assumed that the patient is non-responsive to the treatment.

Alternatively, the responsiveness can be estimated by determining the amount of the compound bound to the alpha-synuclein aggregates. The amount of the compound bound to the alpha-synuclein aggregates can be compared to a control value such as a normal control value, a preclinical control value or a clinical control value. Alternatively, the control value may refer to the control value of subjects known to be responsive to a certain therapy, or the control value may refer to the control value of subjects known to be non-responsive to a certain therapy. The outcome with respect to responsiveness can either be "responsive" to a certain therapy, "non-responsive" to a certain therapy or "response undetermined" to a certain therapy. Response to the therapy may be different for the respective patients.

In yet another embodiment the present invention provides a method, as defined herein, wherein the step of optionally correlating the presence or absence of the compound bound to the alpha-synuclein aggregates, with the presence or absence of the alpha-synuclein aggregates in the sample or specific body part or body area comprises
- determining in which amount of the compound bound to the alpha-synuclein aggregates;
- correlating the amount of the compound bound to the alpha-synuclein aggregates with the amount of the alpha-synuclein aggregates in the sample or specific body part or body area; and
- optionally comparing the amount of the amount of the alpha-synuclein aggregates in the sample or specific body part or body area to a normal control value in a healthy control subject.

The control value can be, e.g., a normal control value, a preclinical control value and/or a clinical control value.

A "healthy control subject" or "healthy volunteer (HV) subject" is a person showing no clinical evidence of neurodegenerative disease.

If in any of the above summarized methods the amount of the compound bound with the alpha-synuclein aggregates is higher than the normal control value, then it can be expected that the patient is suffering from or is likely to from a disease, disorder or abnormality associated with alpha-synuclein aggregates or from a synucleinopathy.

Any of the compounds of formula (III-F) can be used in the above summarized methods.

The specific body part or body area is preferably of a mammal, more preferably of a human, including the full body or partial body area or body part of the patient suspected to contain alpha-synuclein aggregates.

The sample can be selected from tissue or body fluids suspected to contain alpha-synuclein aggregates, the sample being obtained from the patient. Preferably, the tissue is selected from brain tissue, tissue of the central nervous system, and tissue of the eye (such as retinal tissue), more preferably brain tissue. Examples of body fluids include cerebrospinal fluid (CSF) or blood. The sample can be from a peripheral organ such as, but not limited to, the gut. The sample can be obtained from a mammal, more preferably a human. Preferably, the sample is an *in vitro* sample from a patient.

In an *in vivo* method, the specific body part or body area can be brought into contact with a compound of formula (III-F) by administering an effective amount of a compound of formula (III-F) to the patient. The effective amount of a compound of formula (III-F) is an amount which is suitable for allowing the presence or absence of alpha-synuclein aggregates in the specific body part or body area to be determined using the chosen analytical technique.

The step of allowing the compound to bind to the alpha-synuclein aggregates includes allowing sufficient time for the compound of formula (III-F) to bind to the alpha-synuclein aggregates. The amount of time required for binding will depend on the type of test (e.g., *in vitro* or *in vivo)* and can be determined by a person skilled in the field by routine experiments. In an *in vivo* method, the amount of time will depend on the time which is required for the compound to reach the specific body part or body area suspected to contain alpha-synuclein aggregates. The amount of time should not be too extended to avoid washout and/or metabolism of the compound of formula (III-F).

The method of detecting the compound bound to the alpha-synuclein aggregates is not particularly limited and depends, among others, on the type of sample, specific body part or body area and whether the method is an *in vitro* or *in vivo* method. Possible detection methods include, but are not limited to a fluorescence imaging technique or a nuclear imaging technique such as positron emission tomography (PET), single photon emission computed tomography (SPECT), magnetic resonance imaging (MRI), and contrast-enhanced magnetic resonance imaging (MRI). The fluorescence imaging technique and/or nuclear imaging technique can be employed for monitoring and/or visualizing the distribution of the compound of formula (III-F) within the sample or the body. The imaging system is such to provide an image of bound detectable label such as radioisotopes, in particular positron emitters or gamma emitters, as present in the tested sample, the tested specific body part or the tested body area. Preferably, the compound bound to the alpha-synuclein aggregates is detected by an imaging apparatus such as PET or SPECT scanner.

The amount of the compound bound with the alpha-synuclein aggregates can be determined by the visual or quantitative analysis, for example, using PET scan images.

In any of the above methods, steps (a) to (c) and, if present, optional step (d) can be repeated at least one time. The repetition of the steps is particularly useful in the method of collecting data for prognosing, the method of collecting data for monitoring the evolution of the disease, the method of collecting data for monitoring the progression and the method of collecting data for predicting responsiveness. In these methods, it may be expedient to monitor the patient over time and to repeat the above steps after a certain period of time has elapsed. The time interval before the above mentioned steps are repeated can be determined by a physician depending on the severity of the disease, disorder or abnormality associated with alpha-synuclein aggregates or the synucleinopathy.

In a further aspect, the present invention refers to a method of imaging a disease, disorder or abnormality associated with alpha-synuclein aggregates, in a subject, the method comprising the steps:
(a) Administering a compound of the formula (III-F), or a stereoisomer, racemic mixture, pharmaceutically acceptable salt, hydrate, or solvate thereof to the subject;
(b) Allowing the compound to bind to the alpha-synuclein aggregates; and
(c) Detecting the compound bound to the alpha-synuclein aggregates.

In a further aspect, the present invention is directed to a method of imaging a disease, disorder or abnormality associated with alpha-synuclein aggregates, in a subject, the method comprising the steps:
(a) Administering a compound of the formula (III-F), or a stereoisomer, racemic mixture, pharmaceutically acceptable salt, hydrate, or solvate thereof to the subject; and
(b) Imaging the brain of the subject.

The brain of the subject should be imaged when the compound has bound to the alpha-synuclein aggregates,. The compound bound to the alpha-synuclein aggregates can then be imaged in the subject's brain.

In a further aspect, the present invention refers to a method of positron emission tomography (PET) imaging of alpha-synuclein aggregates in a tissue of a subject, the method comprising the steps:
(a) Administering a compound of the formula (III-F), or a stereoisomer, racemic mixture, pharmaceutically acceptable salt, hydrate, or solvate thereof to the subject;
(b) Allowing the compound to penetrate into the tissue of the subject; and
(c) Collecting a positron emission tomography (PET) image of the tissue of the subject;
wherein the tissue is tissue of the central nervous system (CNS), of the eye or brain tissue, preferably wherein the tissue is brain tissue.

The PET imaging should be conducted when the compound has penetrate into the tissue and the compound has bound to the alpha-synuclein aggregates,.

In a further aspect, the present invention is directed a method of detecting a neurological disease, disorder or abnormality associated with alpha-synuclein aggregates in a subject, the method comprising the steps:
(a) Administering a compound of the formula (III-F), or a stereoisomer, racemic mixture, pharmaceutically acceptable salt, hydrate, or solvate thereof to the subject;
(b) Allowing the compound to bind to the alpha-synuclein aggregates; and
(c) Measuring the radioactive signal of the compound, which is bound to the alpha-synclein aggregates.

The radioactive signal, as mentioned herein, is observed when a detectably labelled compound of formula (III-F), which comprises at least one radiolabelled atom, is bound to the alpha-synuclein aggregates.

In a further aspect, the present invention is directed to a method (e.g., an *in vivo* or *in vitro* method) for the detection and/or quantification of alpha-synuclein aggregates in a tissue of a subject, the method comprising the steps:
(a) Contacting the tissue with a compound of the formula (III-F), or a stereoisomer, racemic mixture, pharmaceutically acceptable salt, hydrate, or solvate thereof to the subject;
(b) Allowing the compound to bind to the alpha-synuclein aggregates; and
(c) Detecting and/or quantifying the compound bound to the alpha-synuclein aggregates using positron emission tomography.

In yet another aspect, the present invention refers to a method of the diagnostic imaging of the brain of a subject, the method comprising the steps:
(a) Administering a compound of the formula (III-F), or a stereoisomer, racemic mixture, pharmaceutically acceptable salt, hydrate, or solvate thereof to the subject; and
(b) Obtaining an image of the brain of the subject using positron emission tomography.

In the methods of the present invention, the compound of the formula (III-F), or a stereoisomer, racemic mixture, pharmaceutically acceptable salt, hydrate, or solvate thereof is typically administered in a detectable amount, i.e., an amount which can be detected by the device which is employed in for detecting the compound in the respective method. The amount is not particularly limited and will depend on the compound of the formula (III-F), the type of detectable label, the sensitivity of the respective analytical method and the respective device. The amount can be chosen appropriately by a skilled person.

### RADIOPHARMACEUTICAL PREPARATIONS

The compounds of formula (IV-F), or a stereoisomer, racemic mixture, pharmaceutically acceptable salt, hydrate, or solvate thereof, can also be employed in kits for the preparation of radiopharmaceutical preparations. Due to the radioactive decay, the radiopharmaceuticals are usually prepared immediately before use. The kit typically comprises the compound of formula (IV-F), or a stereoisomer, racemic mixture, pharmaceutically acceptable salt, hydrate, or solvate thereof, and an agent which reacts with the compound of formula (IV-F) to introduce a radioactive label (¹⁸F) into the compound of formula (IV-F), or a stereoisomer, racemic mixture, pharmaceutically acceptable salt, hydrate, or solvate thereof.

The invention is illustrated by the following examples which, however, should not be construed as limiting.

### EXAMPLES

All reagents and solvents were obtained from commercial sources and used without further purification.

*HPLC-UV purity:* A solution of 1 mg/mL of the respective compound is prepared in a 2% aqueous TFA solution and 5 µL is injected on a Ascentis Express 160 A ES-C18, 5 µm, 150 × 4.6 mm column. The respective peak is eluted with a flow rate of 1,5 mL/min, using 0.1% TFA (mobile phase A) and 0,1% TFA in MeCN (mobile phase B) and a linear gradient of 5% to 100% B within 10 min. Detection is performed by UV at 254 nm wavelength.

*HPLC-UV chiral purity:* A solution of approx. 0.5 mg/mL of the respective compound is prepared in MeCN/IPA/5mM NH₄OAc = 3/4/1 (sample filtered if needed) and 10 µL is injected on a Daicel Chiralpak OX-RH, 5 µm, 150 × 4.6 mm column. Peak of precursor S-enantiomer (Rt approx. 7.9 min) is eluted isocratically at 40 °C, with a flow rate of 1 mL/min, using a mixture of MeCN:Water containing 5 mM NH₄OAc at 75:25 per volume ratio. Detection is performed by UV at 254 nm wavelength.

*Identity:* Identity of the respective compound is confirmed by ¹H and ¹³C nuclear magnetic resonance (NMR) spectra obtained with a Bruker AV500 spectrometer, while ESI-MS molecular ion confirmation is obtained using a Thermo Fisher Scientific Surveyor MSQ Plus mass detector in the positive ionization mode. The LC analysis is performed on a XSelect Peptide CSH C18 column, 130 Å, 3.5 µm, 150 × 2.1 mm. For sample preparation 0.5 mg of the respective compound are dissolved in 1 mL MeCN/H₂O (1:1) and the suspension is filtered. After injection, the respective compound is eluted with a flow rate of 0.4 mL/min, using 0.1% HCO₂H (mobile phase B) and 0,1% HCO₂H in MeCN (mobile phase A) and a linear gradient of 5% to 100% A within 15 mins.

### Preparative Example 1

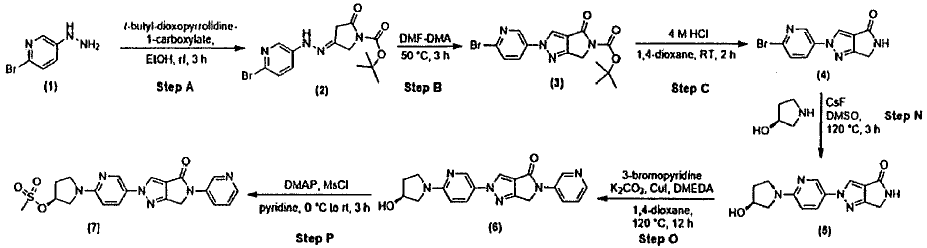

### Step A:

A suspension of 2-bromo-5-hydrazinylpyridine (3.21 g, 17.07 mmol) and tert-butyl 2,4-dioxopyrrolidine-1-carboxylate (3.40 g, 17.07 mmol) in ethanol (150 mL) was refluxed for 3 h and monitored by TLC. The crude product was concentrated under reduced pressure and diluted with dichloromethane and water. The layers were separated and the aqueous layer was extracted twice with dichloromethane. The combined organic layer was dried over Na₂SO₄, filtered and concentrated under reduced pressure. The crude product was purified by flash chromatography (silica, ethyl acetate:hexane = 4:1) to afford (E)-tert-butyl 4-(2-(6-bromopyridin-3-yl)hydrazono)-2-oxopyrrolidine-1-carboxylate as a brown solid (4.97g, 79%).

### Step B:

The compound from step A (3.9 g, 10.56 mmol) was stirred in 1,1-dimethoxy-N,N-dimethylmethanamine (80 mL) at 50°C for 3 h. The reaction was concentrated to approx. 10 mL and ethanol was added. The solid was filtered and washed with small portions of ethanol to afford tert-butyl 2-(6-bromopyridin-3-yl)-4-oxo-4,6-dihydropyrrolo[3,4-c]pyrazole-5(2H)-carboxylate as a light brown powder (2.30 g, 57.4 %).

### Step C:

The compound from step B (1000 mg, 2.64 mmol) was stirred in 4 M HCl in dioxane (37 mL) at room temperature (RT) for 4 h. The solvent was evaporated under reduced pressure and the solid suspended in dichloromethane. A solution of saturated NaHCO₃ was added, and the aqueous phase was extracted twice. The combined organic layers were filtrated to afford 2-(6-bromopyridin-3-yl)-5,6-dihydropyrrolo[3,4-c]pyrazol-4(2H)-one as a beige solid. (682 mg, 93 %)

### Step N:

In a round bottom flask under argon, 2-(6-bromopyridin-3-yl)-5,6-dihydropyrrolo[3,4-c]pyrazo1-4(2H)-one (7.03 g, 25.17 mmol), (S)-pyrrolidin-ol (3.29 g, 37.76 mmol), and cesium fluoride (6.69 g, 50.35 mmol) were mixed in dry DMSO (70 mL). The resulting mixture was flushed with argon and stirred at 120°C for 3 h. The reaction mixture was cooled down and poured into cold water pre-cooled in an ice bath. The resulting suspension was filtered, and the solid rinsed with ice-cold water. A volume of 3 mL of isopropanol was used to triturate the solid directly in the frit to afford the product as a beige solid (6.75 g, 7.23 mmol, 94 %).

### Step O:

In a flask under argon compound from step N (6.26 g, 21.94 mmol), 3-bromopyridine (4.65 mL, 48.3 mmol), potassium carbonate (5.27 g, 87.75 mmol) and copper(I) iodide (1.67 g, 8.78 mmol) were mixed, and the system was flushed with argon. Dioxane (772 mL) and N1,N2-dimethylethane-1,2-diamine (1.87 mL, 17.55 mmol) were added and the mixture was stirred at 110°C for 22 h. The crude product was concentrated under reduced pressure and suspended in water. Ammonia was added until the solution was basic (pH 13). The aqueous layer was extracted twenty times with a solution of DCM/MeOH (9:1). The combined organic layers were dried over Na₂SO₄, filtered, and concentrated to dryness to afford the product as a beige solid (6.36 g, 17.56 mmol, 80 %).

### Step P:

In a vial under argon and cooled to 0°C, (S)-2-(6-(3-hydroxypyrrolidin-1-yl)pyridin-3-yl)-5-(pyridin-3-yl)-5,6-dihydropyrrolo[3,4-c]pyrazol-4(2H)-one (100 mg, 0.276 mmol), and 4-dimethylaminopyridine (337 mg, 2.76 mmol) were mixed in pyridine (4 mL). Mesyl-chloride (0.108 mL, 1.380 mmol) was added, and the mixture was flushed with argon. Pyridine (13 mL) was added and the reaction mixture was transferred to a flask. The reaction mixture was allowed to warm up to RT and stirred for 2 h, after this time 4-dimethylaminopyridine (169 mg, 1.380 mmol) and mesyl-chloride (0.054 mL, 0.690 mmol) were added at 0°C. The reaction was finished after 40 min. Then 0.1 N NaOH in water (5 mL) was added to the mixture to basify it. As it was not basic enough, additional 1N NaOH in water (15 mL) was added. The reaction mixture became brown at pH=14. The solution was poured in cold water and filtered. The solid residue was washed with water until the pH of the water was 7. The obtained solid was dried under high vacuum for 30 mins to afford the compound as an orange solid (86 mg, 0.195 mmol, 70.8%).

It was observed that the product was not stable and was sensitive to temperature, oxygen and light.

### Example 1

### 1st crystallization:

The compound of Preparative Example 1 (2.97 g, 6.7 mmol, purity: 93.7 %) was suspended in ethanol (148 mL) and heated to reflux with an oil bath. DMSO (233 mL) was added at 80°C until a solution was obtained. The oil bath was then removed, and the solution was allowed to cool to room temperature. The flask was sealed and stored at 2 to 8°C overnight. The precipitate was isolated by suction filtration and washed three times with ethanol (1.8 g, 4.1 mmol, 61% yield, HPLC purity 95.5%).

### 2nd crystallization:

The crystallized mesylate precursor from cycle 1 (1.8 g, 4.1 mmol) was suspended in DMSO (60 mL) at room temperature. The suspension was heated to 80°C (a solution was formed at approx. 70°C). At 80°C, ethanol (60 mL) was added and the resulting slightly turbid solution was stirred for 10 min at room temperature. The oil bath was removed, and the solution was allowed to cool to room temperature. The flask was sealed and stored at 2 to 8°C overnight. The precipitate was isolated by suction filtration and washed three times with ethanol. (1.6 g, 3.6 mmol, 89% yield, HPLC purity 96.4%).

### 3rd crystallization:

The crystallized mesylate precursor (1.6 g, 3.6 mmol) from cycle 2 was further crystallized by repeating conditions of cycle 2. (1.5 g, 3.3 mmol, 93% yield, HPLC purity 96.9%).

### Example 2: Stability testing

The compound of Preparative Example 1 was transferred into glass vials and flashed with argon. These vials were stored frozen (-20 °C ± 5 °C), refrigerated (2-8 °C), at room temperature (20-25 °C) both protected and unprotected from light, and at 40 °C. The material was retested by HPLC-UV on a weekly basis for the 1^{st} month, and monthly thereafter. Degradation of the compound of Preparative Example 1 was noticed in all conditions tested. It was however significantly minimized when the material was stored refrigerated at -20 °C and protected from light. The main degradation product initially formed was detected at RT 1.6 min by HPLC-UV and identified by HPLC-ESI-MS as a pyrrole decomposition product generated by elimination of the mesylate group and subsequent aromatization. The pyrrole decomposition product presumably has the following structure

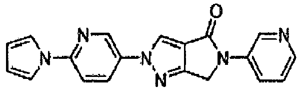

The purified compound of Example 1 was also evaluated in a stress test study at 40 °C. As can be seen from Figure 1, the purification according to the method of the present invention significantly improved the stability.

### Example 3

### 1st crystallization:

1.21 g of the compound of Preparative Example 1 (HPLC purity: 93.2%) were suspended in 40ml DMSO and heated to 80°C. The compound was completely dissolved at 80°C. 40 ml ethanol were added at 80°C and the mixture was stirred at 80°C for 10 min. The stirrer was switched off and the mixture was allowed to cool to RT. The flask was stored at 2 to 8°C over night to complete crystallization. The product was isolated by suction filtration of the still cold suspension and was washed three times with ethanol. Yield: 963 mg, 80%, HPLC purity: 93.6%

### 2nd crystallization:

963 mg were crystallized using the method described above to yield 880 mg, 92%, HPLC purity: 96.1%

### 3rd crystallization:

880 mg were crystallized using the method described above to yield 810 mg, 92%, HPLC purity: 96.7%

### 4th crystallization:

250 mg from material isolated from 3^{rd} crystallization above was suspended in DMSO (8.9 mL) at room temperature. The suspension was heated to 80°C (a solution was formed at approx. 70°C). At 80°C, ethyl acetate (8.9 mL) was added, and the resulting solution was stirred for 10 min at 80°C. The oil bath was removed, and the solution was allowed to cool to room temperature. The flask was sealed and stored at 2 to 8°C overnight. The precipitate was isolated by suction filtration and washed three times with ethyl acetate. (200 mg, 0.45 mmol, 80% yield, HPLC purity 97.6%).

### Example 4

### 1st crystallization:

1.21 g of the compound of Preparative Example 1 (HPLC purity: 93.2%) were suspended in 40ml DMSO and heated to 80°C. The compound was completely dissolved at 80°C. 40 ml ethanol were added at 80°C and the mixture was stirred at 80°C for 10 min. The stirrer was switched off and the mixture was allowed to cool to RT. The flask was stored at 2 to 8°C over night to complete crystallization. The product was isolated by suction filtration of the still cold suspension and was washed three times with ethanol. Yield: 963 mg, 80%, HPLC purity: 93.6%

### 2nd crystallization:

963 mg were crystallized using the method described above to yield 880 mg, 92%, HPLC purity: 96.1%

### 3rd crystallization:

880 mg were crystallized using the method described above to yield 810 mg, 92%, HPLC purity: 96.7%

### 4th crystallization:

250 mg from material isolated from 3^{rd} crystallization above was suspended in DMSO (8.9 mL) at room temperature. The suspension was heated to 80°C (a solution was formed at approx. 70°C). At 80°C, 2-butanone (8.9 mL) was added, and the resulting solution was stirred for 10 min at 80°C. The oil bath was removed, and the solution was allowed to cool to room temperature. The flask was sealed and stored at 2 to 8°C overnight. The precipitate was isolated by suction filtration and washed three times with ethyl acetate. (175 mg, 0.40 mmol, 70% yield, HPLC purity 97.9%).

### Example 5

### 1st crystallization:

14.7g of the compound of Preparative Example 1 (HPLC purity: 92.2%) were suspended in 484ml DMSO and heated to 80°C. The compound was completely dissolved at 80°C. 484 ml ethanol were added at 80°C and the mixture was stirred at 80°C for 10 min. The stirrer was switched off and the mixture was allowed to cool to RT. The flask was stored at 2 to 8°C over night to complete crystallization. The product was isolated by suction filtration of the still cold suspension and was washed three times with 118 ml ethanol. The product was transferred to a flask and dried under high vacuum for three hours. Yield: 12.8g, 29.1 mmol, 87.1%, HPLC purity: 96.2%

### 2nd crystallization:

12.8g were crystallized using the method described above to yield 11.9g, 27.0 mmol, 93%, HPLC purity: 96.9%

### 3rd crystallization:

11.9g were crystallized using the method described above to yield 11.1g, 25.2 mmol, 93.3%, HPLC purity: 97.1%

### 4th crystallization:

11.1g were crystallized using the method described above to yield 10.4g, 23.6 mmol, 93.7%, HPLC purity: 97.3%

### 5th crystallization:

10.4g were crystallized using the method described above to yield 9.9g, 22.5 mmol, 95.2%, HPLC purity: 98.1%.

The overall yield after the cycles of crystallization was 67.4 %.

### Example 6

Comparative compounds of formula (IV-F) in which LG is nosylate or tosylate instead of mesylate were synthesized according to the method of Preparative Example 1. The tosylate compound could not be obtained by this method.

The 5 mg of mesylate compound of Preparative Example 1 and 5 mg of the corresponding nosylate compound were subjected to radiolabelling in the presence of 10 mg Kryptofix[222] and 1.24 mg K₂CO₃ in 0.7 ml DMSO at 140 °C for 10 minutes. The labeling yield of the mesylate compound was 60 %, whereas the labeling yield of the comparative nosylate compound was only 7%.

### Example 7 (Comparative)

The compound of Preparative Example 1 was recrystallized twice using ethyl acetate:DMSO as a solvent system.

### 1st crystallization:

The compound of Preparative Example 1 (325 mg, 0.74 mmol, purity: 93.6 %) was suspended in DMSO (11.6 mL) at room temperature. The suspension was heated to 80°C (a solution was formed at approx. 70°C). At 80°C, ethyl acetate (11.6 mL) was added and the resulting solution was stirred for 10 min at 80°C. The oil bath was removed, and the solution was allowed to cool to room temperature. The flask was sealed and stored at 2 to 8°C overnight. The precipitate was isolated by suction filtration and washed three times with ethyl acetate (156 mg, 0.35 mmol, 47% yield, HPLC purity 94.7%).

### 2nd crystallization:

The crystallized mesylate precursor (156 mg, 0.35 mmol) from cycle 1 was further crystallized by repeating conditions of cycle 1. (50 mg, 0.11 mmol, 32% yield, HPLC purity 94.1%).

As can be seen, the solvent mixture of Example 7 resulted in a much lower purity and yield compared to the solvent mixture which is used in the method of the present invention.

### Example 8 (Comparative)

The compound of Preparative Example 1 was recrystallized twice using 2-butanone: DMSO as a solvent system.

### 1st crystallization:

The compound of Preparative Example 1 (325 mg, 0.74 mmol, purity: 93.6 %) was suspended in DMSO (11.6 mL) at room temperature. The suspension was heated to 80°C (a solution was formed at approx. 70°C). At 80°C, 2-butanone (11.6 mL) was added, and the resulting solution was stirred for 10 min at 80°C. The oil bath was removed, and the solution was allowed to cool to room temperature. The flask was sealed and stored at 2 to 8°C overnight. The precipitate was isolated by suction filtration and washed three times with 2-butanone. (157 mg, 0.36 mmol, 48% yield, HPLC purity 94.2%).

### 2nd crystallization:

The crystallized mesylate precursor (157 mg, 0.36 mmol) from cycle 1 was further crystallized by repeating conditions of cycle 1. (50 mg, 0.11 mmol, 32% yield, HPLC purity 94.4%).

As can be seen, the solvent mixture of Example 8 resulted in a lower purity and much lower yield compared to the solvent mixture which is used in the method of the present invention.

## Claims

1. A method of purifying a compound of formula (IV-F) 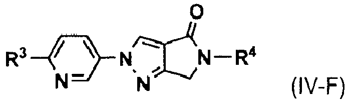 or a stereoisomer, racemic mixture, pharmaceutically acceptable salt, hydrate, or solvate thereof, wherein
R³ is selected from 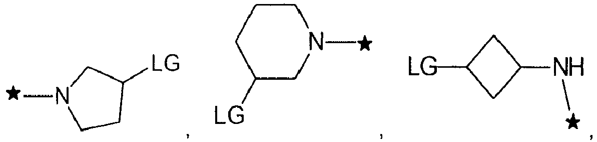 or 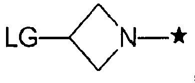 , wherein LG is C₁₋₄ alkyl sulfonate;
R⁴ is an aryl or a 5-membered or 6-membered heteroaryl, wherein R⁴ is selected from: 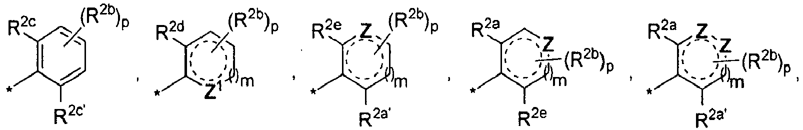 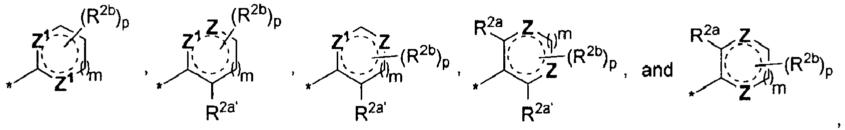 wherein
R^{2a}, R^{2a'} are independently selected from H, or F;
R^{2b} is independently selected from F, -OH, C₁-C₄alkyl, haloC₁-C₄alkyl, -NH₂, -CN, or C₁-C₄alkoxy;
R^{2c}, R^{2c'} are independently selected from H, F, OH, OCH₃, or CH₃;
R^{2d} is selected from H, F, or -OH;
R^{2e} is selected from H, OH, CH₃, or F;
Z is independently N, NH, N(C₁-C₄alkyl), N(haloC₁-C₄alkyl), O, or S;
Z¹ is independently N, NH, O, or S;
p is 0, 1 or 2;
m is 0 or 1;
as valency permits, is a combination of single and double bonds; and
* is the position of bonding;
wherein the method comprises
(i) dissolving a crude compound of formula (IV-F) in DMSO and ethanol to obtain a solution; and
(ii) conducting crystallization from the solution to obtain a purified compound of formula (IV-F).

2. The method according to claim 1, wherein the compound of formula (IV-F) is 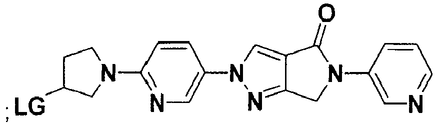 or a pharmaceutically acceptable salt, hydrate, or solvate thereof.

3. The method according to claim 1 or 2, wherein **LG** is mesylate.

4. The method according to any one of claims 1 to 3, which further comprises the step of
(iii) isolating the purified compound of formula (IV-F) from the solution, and optionally washing and drying the isolated purified compound of formula (IV-F).

5. The method according to any one of claims 1 to 4, wherein step (i), step (ii) and optionally step (iii) are conducted at least twice, preferably at least three times, more preferably at least four times, even more preferably at least five times.

6. The method according to any one of claims 1 to 5, wherein step (i) comprises
mixing the crude compound of formula (IV-F) with DMSO and then adding ethanol to the obtained mixture.

7. The method according to any one of claims 1 to 6, wherein the ratio (v:v) of DMSO to ethanol is in the range of about 5:1 to about 0.1:1, preferably about 4:1 to about 0.5:1, more preferably about 3:1 to about 0.5:1, even more preferably about 2:1 to about 0.75:1.

8. The method according to any one of claims 1 to 7, wherein the crude compound of formula (IV-F) is dissolved at a temperature in the range of about 50 to about 90 °C, preferably about 60 to about 90 °C, more preferably about 65 to about 85 °C.

9. The method according to any one of claims 1 to 8, wherein the crystallization is conducted at a temperature in the range of about 0 °C to about 40 °C, preferably about 0 °C to about 40 °C, more preferably about 0 °C to about 30 °C.

10. The method according to any one of claims 1 to 9, which further comprises after step (ii) or, if present, optional step (iii)
(iv) dissolving the compound of formula (IV-F) in DMSO and 2-butanone or ethyl acetate to obtain a solution; and
(v) conducting crystallization from the solution to obtain a purified compound of formula (IV-F).

11. The method of any one of claims 1 to 10, wherein the purified compound of formula (IV-F) has a purity of at least 97 %.

12. A compound obtainable by the method according to any one of claims 1 to 11.

13. A kit for preparing a radiopharmaceutical preparation, wherein the kit comprises a sealed vial containing at least one compound as defined in claim 12.

14. The kit according to claim 13, wherein the radiopharmaceutical preparation is for use in the diagnostics of a disease, disorder or abnormality associated with alpha-synuclein aggregates or a predisposition therefor, wherein the disease, disorder or abnormality is optionally selected from Parkinson's disease (including sporadic, familial with alpha-synuclein mutations, familial with mutations other than alpha-synuclein, pure autonomic failure or Lewy body dysphagia), SNCA duplication carrier, Lewy Body dementia (LBD), dementia with Lewy bodies (DLB) (including "pure" Lewy body dementia), Parkinson's disease dementia (PDD), diffuse Lewy body disease (DLBD), Alzheimer's disease, sporadic Alzheimer's disease, familial Alzheimer's disease with APP mutations, familial Alzheimer's disease with PS-1, PS-2 or other mutations, familial British dementia, Lewy body variant of Alzheimer's disease, Down syndrome, multiple system atrophy (MSA) (including Shy-Drager syndrome, striatonigral degeneration or olivopontocerebellar atrophy), traumatic brain injury, chronic traumatic encephalopathy, dementia puglistica, tauopathies (including Pick's disease, frontotemporal dementia, progressive supranuclear palsy, corticobasal degeneration, Niemann-Pick type C1 disease, frontotemporal dementia with Parkinsonism linked to chromosome 17), Creutzfeldt-Jakob disease, Huntington's disease, motor neuron disease, amyotrophic lateral sclerosis (including sporadic, familial or ALS-dementia complex of Guam), neuroaxonal dystrophy, neurodegeneration with brain iron accumulation type 1 (including Hallervorden-Spatz syndrome), prion diseases, ataxia telangiectatica, Meige's syndrome, subacute sclerosing panencephalitis, Gerstmann-Straussler-Scheinker disease, inclusion-body myositis, Gaucher disease, Krabbe disease as well as other lysosomal storage disorders (including Kufor-Rakeb syndrome and Sanfilippo syndrome) and rapid eye movement (REM) sleep behavior disorder, wherein the disease is preferably selected from Parkinson's disease, multiple system atrophy, dementia with Lewy bodies, Parkinson's disease dementia, SNCA duplication carrier, and Alzheimer's disease, more preferably wherein the disease is multiple system atrophy.

15. The kit according to claim 13, wherein the radiopharmaceutical preparation is for use in the imaging of alpha-synuclein aggregates, wherein the imaging is preferably conducted by positron emission tomography.

16. The kit according to claim 13, wherein the radiopharmaceutical preparation is for use is for *in vitro* imaging, *ex vivo* imaging, or *in vivo* imaging, preferably the use is for *in vivo* imaging, more preferably the use is for brain imaging.

17. A method of preparing a detectably labelled compound of formula (III-F) 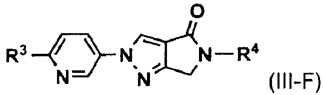 or a stereoisomer, racemic mixture, pharmaceutically acceptable salt, hydrate, or solvate thereof,
wherein
R³ is selected from 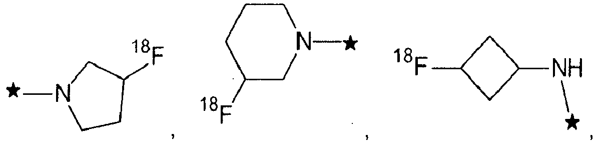 or 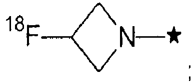
R⁴ is an aryl or a 5-membered or 6-membered heteroaryl, wherein R⁴ is selected from: 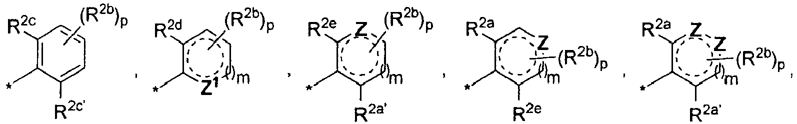 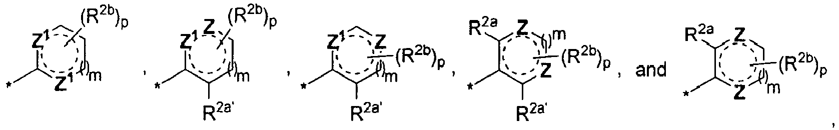 wherein
R^{2a}, R^{2a'} are independently selected from H, or F;
R^{2b} is independently selected from F, -OH, C₁-C₄alkyl, haloC₁-C₄alkyl, -NH₂, -CN, or C₁-C₄alkoxy;
R^{2c}, R^{2c'} are independently selected from H, F, OH, OCH₃, or CH₃;
R^{2d} is selected from H, F, or -OH;
R^{2e} is selected from H, OH, CH₃, or F;
Z is independently N, NH, N(C₁-C₄alkyl), N(haloC₁-C₄alkyl), O, or S;
Z¹ is independently N, NH, O, or S;
p is 0, 1 or 2;
m is 0 or 1;
as valency permits, is a combination of single and double bonds; and
* is the position of bonding;
wherein the method comprises
reacting the compound according to claim 12 or the compound prepared by the method according to any one of claims 1 to 11 with a ¹⁸F-fluorinating agent, so that LG is replaced by ¹⁸F.

18. The method according to claim 17, wherein the ¹⁸F-fluorinating agent is selected from K¹⁸F, Rb¹⁸F, Cs¹⁸F, Na¹⁸F, Rb¹⁸F, Kryptofix[222]K¹⁸F, tetra(C₁₋₆ alkyl) ammonium salt of ¹⁸F, and tetrabutylammonium [¹⁸F]fluoride.

19. The method according to claim 17 or 18, wherein the detectably labelled compound of formula (III-F) is for use in the diagnostics of a disease, disorder or abnormality associated with alpha-synuclein aggregates or a predisposition therefor, wherein the disease, disorder or abnormality is optionally selected from Parkinson's disease (including sporadic, familial with alpha-synuclein mutations, familial with mutations other than alpha-synuclein, pure autonomic failure or Lewy body dysphagia), SNCA duplication carrier, Lewy Body dementia (LBD), dementia with Lewy bodies (DLB) (including "pure" Lewy body dementia), Parkinson's disease dementia (PDD), diffuse Lewy body disease (DLBD), Alzheimer's disease, sporadic Alzheimer's disease, familial Alzheimer's disease with APP mutations, familial Alzheimer's disease with PS-1, PS-2 or other mutations, familial British dementia, Lewy body variant of Alzheimer's disease, Down syndrome, multiple system atrophy (MSA) (including Shy-Drager syndrome, striatonigral degeneration or olivopontocerebellar atrophy), traumatic brain injury, chronic traumatic encephalopathy, dementia puglistica, tauopathies (including Pick's disease, frontotemporal dementia, progressive supranuclear palsy, corticobasal degeneration, Niemann-Pick type C1 disease, frontotemporal dementia with Parkinsonism linked to chromosome 17), Creutzfeldt-Jakob disease, Huntington's disease, motor neuron disease, amyotrophic lateral sclerosis (including sporadic, familial or ALS-dementia complex of Guam), neuroaxonal dystrophy, neurodegeneration with brain iron accumulation type 1 (including Hallervorden-Spatz syndrome), prion diseases, ataxia telangiectatica, Meige's syndrome, subacute sclerosing panencephalitis, Gerstmann-Straussler-Scheinker disease, inclusion-body myositis, Gaucher disease, Krabbe disease as well as other lysosomal storage disorders (including Kufor-Rakeb syndrome and Sanfilippo syndrome) and rapid eye movement (REM) sleep behavior disorder, wherein the disease is preferably selected from Parkinson's disease, multiple system atrophy, dementia with Lewy bodies, Parkinson's disease dementia, SNCA duplication carrier, and Alzheimer's disease, more preferably wherein the disease is multiple system atrophy.

20. The method according to claim 17 or 18, wherein the detectably labelled compound of formula (III-F) is for use in the imaging of alpha-synuclein aggregates, wherein the imaging is preferably conducted by positron emission tomography.

21. The method according to claim 17 or 18, wherein the detectably labelled compound of formula (III-F) is for use is for *in vitro* imaging, *ex vivo* imaging, or *in vivo* imaging, preferably the use is for *in vivo* imaging, more preferably the use is for brain imaging.
